# EUROPEAN PATENT SPECIFICATION

(11) **EP 1 716 233 B1**
(45) Date of publication and mention of the grant of the patent: **26.08.2009**
(21) Application number: 05700620.7
(22) Date of filing: 28.01.2005
(51) Int. Cl.: C12N 15/10, C12N 15/62, C12N 15/65, C12N 5/10, C12Q 1/68

(54) **REGULATED STOP CODON READTHROUGH**
GESTEUERTES ÜBERLESEN VON STOPCODONS
TRANSLECTURE REGULEE D'UN CODON D'ARRET

(30) Priority: 30.01.2004 US 540820 P; 29.11.2004 US 631306 P
(43) Date of publication of application: 02.11.2006
(73) Proprietor: Maxygen Holdings Ltd., Grand Cayman (KY); Maxygen Aps, 2970 Hoersholm (DK)
(72) Inventor: BOUQUIN, Thomas, DK-2980 Kokkedal (DK)
(74) Representative: Hallybone, Huw George
(86) International application number: PCT/DK2005/000070
(87) International publication number: WO 2005/073375

(56) References cited:
- EP-A- 1 162 270
- WO-A-01/44516
- WO-A-02/34906
- WO-A-03/014361
- US-A1- 2003 124 555
- ABO TATSUHIKO ET AL: "SsrA-mediated protein tagging in the presence of miscoding drugs and its physiological role in Escherichia coli" GENES TO CELLS, vol. 7, no. 7, July 2002 (2002-07), pages 629-638, XP002333269 ISSN: 1356-9597
- ARNER E S J ET AL: "High-level expression in Escherichia coli of selenocysteine-containing rat thioredoxin reductase utilizing gene fusions with engineered bacterial-type SECIS elements and co-expression with the selA, selB and selC genes" JOURNAL OF MOLECULAR BIOLOGY, LONDON, GB, vol. 292, no. 5, 8 October 1999 (1999-10-08), pages 1003-1016, XP004457250 ISSN: 0022-2836

## Description

### FIELD OF THE INVENTION

The present invention relates to various methods based on selective suppression of stop codons during protein translation, primarily based on use of an aminoglycoside antibiotic, including methods for alternative production of soluble or membrane-bound proteins from the same cell, for selection of cell clones or cells, and for evaluation of protein expression.

### BACKGROUND OF THE INVENTION

While the anticodons of aminoacyl transfer RNAs (tRNAs) recognize sense codons, leading to the incorporation of a specific amino acid, there are no eukaryotic tRNAs with anticodons that match any of the three stop (nonsense) codons UAA, UGA and UAG. Translation termination occurs when a stop codon enters the A site of the ribosome and is controlled essentially by the release factor eRF1, whose function is modulated by the GTPase eRF3 (Stansfield, 1995; Zhouravleva, 1995). Translation termination is normally a highly efficient process. However, the misincorporation of an amino acid at the stop codon, also termed suppression or translational readthrough, can be influenced by several parameters, among which the local sequence context surrounding the stop codon seems to play a major role. The importance of the nucleotide immediately downstream the nonsense codon has been assessed in *in vitro* translational assays and it has been confirmed that the actual translational termination efficiency is strongly dependent on a tetranucleotide sequence (Manuvakhova et al., 2000).

The antibiotics belonging to the group of aminoglycosides have long been known to interfere with the decoding center of the ribosomal RNA (rRNA). These antibiotics cause misreading of the RNA code and can allow the insertion of alternative amino acids at the site of a stop codon (Palmer et al., 1979). Depending upon the dose, these drugs may inhibit protein synthesis. These observations have raised the possibility that diseases caused by nonsense mutations could be treated by aminoglycoside antibiotics. Some researchers have used this property of aminoglycosides in cell cultures or transgenic animals exhibiting nonsense codons within a structural gene to allow the translational machinery to translate the full mRNA, and thus complement the mutation. Using cultured mammalian cells, Burke and Mogg (1985) showed that the aminoglycoside antibiotics paromomycin and G-418 could partially restore the synthesis of a full-size protein from a mutant gene with a premature UAG mutation. Later, G-418 and gentamicin were shown to restore the expression of the cystic fibrosis transmembrane conductance regulator (CFTR) protein in a cell line carrying a nonsense mutation in CFTR (Bedwell et al., 1997; Howard et al., 1996). A similar study has been done in mutant mice exhibiting a premature stop codon in the dystrophin gene (Barton-Davis et al., 1999). These observations indicate that aminoglycosides are efficient both in cultured cells and in whole organisms to promote translational readthrough.

US 2002/0086427 A1 discloses an inducible eukaryotic expression system in which the expression of a desired gene can be activated or deactivated at the level of gene translation via an inducible signal. This is accomplished by introducing a mutation into the coding sequence of the gene of interest that causes a decrease or alteration of translation, e.g. a stop codon, and by contacting the eukaryotic cell containing the mutated gene of interest with an agent that suppresses the effect of the mutation, e.g. an aminoglycoside.

WO 03/014361 discloses a method for selection of single cell clones using stop codon-dependent translational coupling of marker gene expression with gene of interest expression, resulting in two recombinant gene products, a product encoded by the gene of interest and a fusion protein comprising the gene of interest combined with the selectable marker gene. The marker gene is e.g. a drug resistance gene or a reporter gene such as the GFP (green fluorescent protein) gene. The method may include use of a stop codon suppression mechanism, e.g. a SECIS element (selenocystein insertion sequence) to obtain insertion of the amino acid selenocystein at an UGA stop codon.

WO 03/099996 describes a method of selecting a cell producing a secreted polypeptide by providing a cell population that comprises a cell comprising a heterologous nucleic acid encoding a secreted polypeptide, contacting the cell population with a compound that specifically binds to the secreted polypeptide, detecting the binding of the compound to the secreted polypeptide on the surface of the cell, and selecting the cell based upon the presence or amount of the compound bound to the secreted polypeptide on the surface of the cell.

WO 01/44516 discloses screening methods that involve suppression of a stop codon in an expression cassette that comprises a reporter peptide downstream of a polypeptide of interest.

It has now been found that aminoglycoside antibiotics may be used to selectively obtain translational readthrough for e.g. alternative production of soluble and membrane-bound or otherwise tagged or marked forms of a recombinant protein from the same vector. This finding has important implications for providing a variety of improved and advantageous methods for selection and evaluation of cell clones or individual cells, and for evaluation of protein expression.

### SUMMARY OF THE INVENTION

In its broadest aspect, the invention relates td various methods, sometimes referred to below as "Regulated Readthrough", for screening or selecting cells expressing a polypeptide of interest, as well as for producing a polypeptide of interest from a selected cell; where the cells comprise an expression cassette comprising a gene of interest and a sequence encoding one or more of a cell membrane anchoring peptide, a reporter peptide and an epitope tag, and further at least one stop codon downstream of the sequence encoding the polypeptide of interest.

In one general aspect, the invention relates to methods for screening or selecting cells expressing a desired level of a polypeptide of interest, or for evaluating recombinant polypeptide expression in a population of cells, where the cells comprise an expression cassette comprising, in sequence, a coding sequence for a polypeptide of interest, a stop codon, and a coding sequence for a cell membrane anchoring peptide.

A particular embodiment of this aspect of the invention relates to a method for screening or selecting cells expressing a desired level of a polypeptide of interest, comprising:
a) providing a plurality of eukaryotic cells each comprising an expression cassette comprising a first polynucleotide encoding the polypeptide of interest, at least one stop codon downstream of the first polynucleotide, and a second polynucleotide encoding a cell membrane anchoring peptide downstream of the stop codon;
b) cultivating the cells in the presence of a termination suppression agent under conditions that allow expression of the polypeptide of interest, wherein the termination suppression agent is an aminoglycoside antibiotic; and
c) using flow cytometry to select at least one cell expressing the polypeptide of interest fused to a cell membrane anchoring peptide.

Another particular embodiment of this aspect of the invention relates to a method for evaluating recombinant protein expression in a population of cells, comprising:
a) providing a plurality of eukaryotic cells each comprising an expression cassette comprising a first polynucleotide encoding a recombinant polypeptide, at least one stop codon downstream of the first polynucleotide, and a second polynucleotide encoding a cell membrane anchoring peptide downstream of the stop codon;
b) cultivating the cells in the presence of a termination suppression agent under conditions that allow expression of a fusion protein comprising the recombinant polypeptide and the cell membrane anchoring peptide, wherein the termination suppression agent is an aminoglycoside antibiotic; and
c) sorting the cells by flow cytometry to select at least one cell expressing the fusion protein at a desired level and/or with a desired uniformity.

In another embodiment, the invention relates to a method for screening or selecting at least one cell expressing a polypeptide with a desired binding affinity to a ligand from cells expressing a library of polypeptide variants, comprising:
a) providing a plurality of eukaryotic of cells each comprising an expression cassette comprising a first polynucleotide encoding a polypeptide variant, at least one stop codon downstream of the first polynucleotide, and a second polynucleotide encoding a cell membrane anchoring peptide downstream of the stop codon;
b) cultivating the cells in the presence of a termination suppression agent under conditions that allow expression of the polypeptide variant, wherein the termination suppression agent is an aminoglycoside antibiotic; and
c) using flow cytometry to select at least one cell expressing the polypeptide variant fused to a cell membrane anchoring peptide based on binding affinity of said polypeptide variant to said ligand.

A second general aspect of the invention relates to methods that allow alternate expression of different polypeptides from a single cell or cell line, for example i) a soluble polypeptide or ii) a membrane-bound polypeptide; or i) a membrane-bound, untagged polypeptide or ii) a membrane-bound, tagged polypeptide.

A particular embodiment of this aspect of the invention relates to a method for alternately expressing either i) a soluble polypeptide or ii) a membrane-bound polypeptide from a single cell or cell line, comprising:
a) providing a plurality of eukaryotic cells each comprising an expression cassette comprising a first polynucleotide encoding the polypeptide of interest, at least one stop codon downstream of the first polynucleotide, and a second polynucleotide encoding a cell membrane anchoring peptide downstream of the stop codon;
b) cultivating the cells in the presence of a termination suppression agent under conditions that allow expression of the polypeptide of interest, wherein the termination suppression agent is an aminoglycoside antibiotic;
c) using flow cytometry to select at least one cell expressing the polypeptide of interest fused to a cell membrane anchoring peptide ; and
d) cultivating said selected cell in the absence of a termination suppression agent to obtain expression of the polypeptide of interest as a soluble polypeptide.

A further embodiment of this aspect of the invention relates to a method for alternately expressing i) a membrane-bound, untagged polypeptide or ii) a membrane-bound tagged polypeptide from a single cell or cell line, comprising:
a) providing a plurality of eukaryotic cells each comprising an expression cassette comprising a first polynucleotide encoding the polypeptide of interest and a cell membrane anchoring peptide, at least one stop codon downstream of the first polynucleotide, and a second polynucleotide encoding a reporter peptide or an epitope tag downstream of the stop codon;
b) cultivating the cells in the presence of a termination suppression agent under conditions that allow expression of the polypeptide of interest and the cell membrane anchoring peptide ; wherein the termination suppression agent is an aminoglycoside antibiotic
c) using flow cytometry to select at least one cell expressing a fusion protein comprising the polypeptide of interest, the cell membrane anchoring peptide, and a reporter peptide or an epitope tag; and
d) cultivating said selected cell in the absence of a termination suppression agent to obtain expression of a protein comprising the polypeptide of interest in membrane-bound form without the reporter peptide or epitope tag.

There are also disclosed herein methods suitable for use as alternatives to conventional antibiotic-based selection of cells transformed with a gene of interest, whereby the resulting selected cells may be used for production of a polypeptide of interest without undesired expression of an antibiotic resistance gene. In one embodiment, this disclosure relates to a method for screening or selecting cells expressing a polypeptide of interest from a population of cells, comprising:
a) transfecting a population of cells with an expression cassette comprising, in sequence, a gene of interest, at least one stop codon, and a cell targeting peptide, wherein the expression cassette does not comprise an antibiotic resistance gene;
b) cultivating the transfected population of cells in the presence of a termination suppression agent; and
c) selecting at least one cell expressing the polypeptide of interest fused to a cell targeting peptide.

In another embodiment, a method is disclosed in which antibiotic resistance is used for selection or screening purposes in the presence of an aminoglycoside antibiotic and a non-aminoglycoside antibiotic, but where the selected cells do not express the antibiotic resistance gene under normal production conditions in the absence of an aminoglycoside antibiotic. This embodiment relates to a method for screening or selecting cells expressing a polypeptide of interest from a population of cells, comprising:
a) transfecting a population of cells with an expression cassette comprising, in sequence, a gene of interest, at least one stop codon, and an antibiotic resistance gene, wherein the antibiotic resistance gene provides resistance to a non-aminoglycoside antibiotic;
b) cultivating the transfected population of cells in the presence of an aminoglycoside antibiotic and the non-aminoglycoside antibiotic; and
c) selecting at least one cell which is able to grow in the presence of the non-aminoglycoside antibiotic.

In a further aspect, there is disclosed a method for screening or selecting cell clones expressing a high level of a polypeptide of interest, but where use of an aminoglycoside is unnecessary. This method comprises the steps of:
a) providing a plurality of cells each comprising an expression cassette comprising a first polynucleotide encoding the polypeptide, at least one stop codon downstream of the first polynucleotide, and a second polynucleotide encoding a cell membrane anchoring peptide, a reporter peptide or an epitope tag downstream of the stop codon;
b) cultivating the cells under conditions that allow expression of the polypeptide; and
c) selecting at least one cell expressing the polypeptide fused to a cell membrane anchoring peptide.

A still further aspect of the disclosure relates to a method for producing a polypeptide, comprising cultivating a cell line obtained by any of the methods described herein, wherein the cell line is cultivated in the absence of an aminoglycoside antibiotic to allow expression of the polypeptide, and isolating said polypeptide, e.g. wherein the polypeptide is a soluble polypeptide that is secreted into a culture medium, and the polypeptide is isolated from said medium.

### DESCRIPTION OF THE DRAWINGS

Figure 1 shows the details of the vector *pLenti6-PC-GPI.*
Figure 2 shows the details of the vector *pLenti6-PC-UAAC-GPI.*
Figure 3 shows the details of the vector *pLenti6-PC-UGAC-GPI.*
Figure 4 shows the results of FACS analysis of cell surface expression of protein C (PC) and a GPI anchor with or without a stop codon and in the presence of different amounts of the aminoglycoside antibiotic G-418.
Figure 5 shows the results of FACS sorting and analyses of transgenic cell lines harboring the PC-UAAC-GPI construct, and that have been treated (B) or not treated (A) with G-418. Cells whose fluorescence was included in either gate P2 or P3 of (B) were individually sorted and grown further prior to FACS analysis of membrane-anchored PC (C).
Figure 6 shows a comparison of protein C (PC) activity of 26 individual clones compared to the relative fluorescence of the respective clones determined by FACS.
Figure 7 shows the details of the vector *Retro-IFN-UGAG.*
Figure 8 shows the results of FACS analysis of three different clones for uniformity of recombinant protein expression within the cell populations.
Figure 9 shows the details of the vector *pCDNA6-FVII-UAA-EGFPd.*
Figure 10 shows the details of the vector *pCDNA6-ARI-UAA-V5.*
Figure 11 shows the details of the vector *pCDNA6-FVII-UAA-GPI.*
Figure 12 shows the results of FACS sorting of control CHO-K1 cells (A) and CHO-K1 cells transfected to express a FVII-GPI fusion protein (B) using selection based on aminoglycoside-mediated translational readthrough with G-418 in the absence of an antibiotic resistance gene.
Figure 13 shows the results of a second round of FACS sorting of non-transfected CHO-K1 cells as a control (A) and transfected CHO-K1 cells (B), where the transfected cells were those selected as positive for expression of the FVII-GPI fusion protein as shown in Figure 12 (B) and grown for 10 days prior to analysis.
Figure 14 shows the DNA and amino acid sequence of the PC-GPI cassette (SEQ ID NO:1), including the native PC signal peptide, in the construct of Figure 1 (bold: DNA and amino acid sequence of the GPI anchor; italics: stop codons).
Figure 15 shows the DNA and amino acid sequence of the PC-UAAC-GPI-4Stop cassette (SEQ ID NO:2), including the native PC signal peptide, in the construct of Figure 2 (underlined + italics: readthrough stop codon; bold: DNA and amino acid sequence of the GPI anchor; italics: stop codons).
Figure 16 shows the DNA and amino acid sequence of the PC-UGAC-GPI-4Stop cassette (SEQ ID NO:3), including the native PC signal peptide, in the construct of Figure 3 (underlined + italics: readthrough stop codon; bold: DNA and amino acid sequence of the GPI anchor; italics: stop codons).
Figure 17 shows the DNA and amino acid sequence of the FVII-UAA-GPI cassette (SEQ ID NO:4), including the native FVII signal peptide, in the construct of Figure 11 (underlined + italics: readthrough stop codon; bold: DNA and amino acid sequence of the GPI anchor; italics: stop codons).
Figure 18 shows the DNA and amino acid sequence of the IFN-UGAG cassette (SEQ ID NO:5), including a synthetic signal peptide, in the construct of Figure 7 (underlined: DNA sequence of mature human IFNα-21b; boxed: DNA sequence of E-tag and S-tag; underlined + italics: readthrough stop codon; shaded: DNA sequence of V5 epitope; bold: DNA and amino acid sequence of the GPI anchor; italics: stop codons).
Figure 19 shows the details of the vector *Retro-HC Lib*
Figure 20 shows the details of the vector *Retro-LC Lib*
Figure 21 shows the details of the vector *pB205.*
Figure 22 shows the DNA and amino acid sequence of the FVII-UAA-GPI cassette (SEQ ID NO:6) in the construct of Figure 21, including the native FVII signal peptide and a modified human FVII sequence with the amino acid substitutions P10Q, K32E, A34E, R36E, T106N and V253N compared to wild-type human FVII (underlined + italics: readthrough stop codon; bold: DNA and amino acid sequence of the GPI anchor; italics: stop codons).
Figure 23 shows the results of serum-free production of soluble recombinant FVII in CHO-K1 clones obtained using "classical" limited dilution cloning compared to production in clones selected using the Regulated Readthrough approach of the invention in conjunction with FACS analysis.

### DETAILED DISCLOSURE OF THE INVENTION

This invention provides, in one embodiment, a system that permits the efficient selection of cell lines expressing high levels of recombinant proteins by using Fluorescence-Activated Cell Sorting (FACS, also known as flow cytometry) and that relies on the property of aminoglycoside antibiotics to promote translational readthrough. The expression cassette is, for example, composed of a recombinant gene of interest (GOI) to be expressed into host cells, followed by a stop codon and a cell membrane anchoring signal. Any one of the three stop codons (UAA, UAG and UGA) in various tetranucleotide contexts can be chosen, depending on the background levels of suppression that are desired, as well as aminoglycoside-dependent inducibility and maximal readthrough levels upon aminoglycoside treatment. In the presence of aminoglycosides, translational readthrough is promoted and a subset of recombinant protein is produced as the recombinant protein fused to the cell membrane anchor signal. As a result, this fusion protein is displayed at the external surface of host cells, and cells displaying high levels of membrane-anchored recombinant protein can be selected by FACS. After cell sorting, cells are cultivated in the absence of aminoglycoside to allow efficient translational termination and production of high levels of soluble recombinant protein.

In another embodiment of the invention, the membrane anchoring signal can be replaced by a reporter gene such as the Green Fluorescent Protein (GFP) or an epitope tag such as the V5 epitope. In the presence of aminoglycosides, translational readthrough is promoted and as a result, a tagged version of the recombinant protein is produced. This allows the easy detection or quantification of recombinant protein expression by western blots or ELISA for example. If only production of native recombinant protein is desired, cells are grown in the absence of aminoglycosides to allow efficient translational termination. Furthermore, if the recombinant protein is a membrane-anchored protein, such as some hormone receptors, the aminoglycoside-mediated readthrough allows sorting of cell lines by FACS using detection antibodies targeted against the reporter gene or epitope. After cell sorting, the aminoglycoside antibiotic is removed from the culture medium to allow the production of untagged recombinant protein.

In another embodiment of the invention, both a reporter gene (or an epitope) and a membrane anchoring signal are translationally fused to the GOI that is followed by a stop codon. The resulting expression cassette (GOI-stop codon-reporter gene-membrane anchoring signal) typically allows efficient FACS-based selection of aminoglycoside-treated cells expressing high levels of recombinant protein because the fusion protein is targeted to the cell membrane. Additionally, the reporter protein or epitope tag, which is downstream of the termination signal, can be used as a target for specific antibodies during the FACS sorting. Alternatively, the reporter protein can be a protein exhibiting natural fluorescent properties (e.g. GFP). When soluble recombinant protein expression is desired, aminoglycosides are removed from the culture medium to allow efficient translational termination. As a result, the native recombinant protein alone may be produced from the same cell or vector used to produce the anchored or tagged version of the polypeptide of interest.

### Definitions

Unless otherwise defined herein or below in the remainder of the specification, all technical and scientific terms used herein have the same meaning as commonly understood by those of ordinary skill in the art to which the present invention belongs.

A "nucleic acid sequence", "polynucleotide sequence" or "polynucleotide" is a nucleic acid (which is a polymer of nucleotides (A,C,T,U,G, etc. or naturally occurring or artificial nucleotide analogues)) or a character string representing a nucleic acid, depending on context. Either the given nucleic acid sequence or the complementary nucleic acid sequence can be determined from any specified polynucleotide sequence.

Similarly, an "amino acid sequence" is a polymer of amino acids (a protein, polypeptide, etc.) or a character string representing an amino acid polymer, depending on context. Either the given nucleic acid or the complementary nucleic acid can be determined from any specified polynucleotide sequence.

The terms "protein", "peptide" or "polypeptide" may be used interchangeably herein to refer to polymers of amino acids, without any of these terms being limited to an amino acid sequence of a particular length. The terms "protein of interest" or "polypeptide of interest" may similarly be used interchangeably in the present context. These terms are intended to include not only full-length proteins but also e.g. fragments or truncated versions, variants, domains, etc. of any given protein or polypeptide. Similarly, the term "peptide" as used herein includes full-length proteins as well as e.g. shorter peptides of any given length depending on the context.

The "population of cells" in the context of the present invention may be any population of any type of cell, in particular eukaryotic cells. The population may comprise cells expressing a library of polypeptides, e.g. a naive antibody library or a library of polypeptide variants where the aim is to identify antibodies or polypeptide variants in the library having a desired binding affinity, or it may comprise a collection of cell clones where the aim is to e.g. identify clones having a high and uniform expression level of a polypeptide of interest. For cell populations that express a library of polypeptides, these may for example be a naïve antibody library, an antibody library obtained via immunization with a target of interest, or a library of an antibody or non-antibody polypeptide of interest that has been subjected to mutagenesis. In the case of mutagenesis libraries, mutagenesis may be performed by any method known in the art. One preferred general mutagenesis method is DNA shuffling or directed evolution; see, for example, Kurtzman et al. (2001) for a review of directed protein evolution as applied to therapeutic proteins, and Whalen et al. (2001) for a review of DNA shuffling as applied to vaccines.

"Selecting" or "screening" refers to identifying one or more cells from a population of cells, wherein the one or more cells fulfill one or more predetermined selection criteria as determined by standard methods known to persons skilled in the art. For example, selection or screening may be performed using FACS or another fluorescence-based method, ELISA or another affinity-based method, or by means of a radioactivity-based method. Cells that are identified as a result of the screening/selection procedure will generally be isolated from non-selected cells of the original cell population, e.g. for use in one or more additional rounds of selection, optionally including (further) mutagenesis, for additional qualitative or quantitative analysis, or for use e.g. in development of a cell line for protein production. In the present specification, the terms "selecting" and "screening" are generally used interchangeably.

In the method of the invention for screening or selecting cells expressing a polypeptide with a desired binding affinity to a ligand from cells expressing a library of polypeptide variants, the ligand may be any molecule that binds to the polypeptide of interest, including both polypeptides and non-peptide molecules ("small molecules"). In the case of polypeptide ligands, the ligand may be any kind of polypeptide for which it is desired to optimize binding to the polypeptide of interest, including a receptor. For example, when the polypeptide of interest is an interferon alpha, the "ligand" in this context may be the interferon alpha receptor 1 or 2, even though these receptors would not normally be considered to be a "ligand". One particularly interesting use for this method of the invention is for screening of antibody libraries based on binding of antibodies to a target antigen.

The polypeptide of interest is not limited to any particular protein or group of proteins, but may on the contrary be any protein, of any function or origin, which one desires to select and/or express by the methods described herein. The polypeptide of interest may thus be a therapeutic protein such as a cytokine, an antibody, a hormone or a therapeutic enzyme. Alternatively, the polypeptide of interest may e.g. be an industrial enzyme.

The polypeptide of interest can be a mature protein or a precursor form thereof, or a functional fragment thereof that essentially has retained a biological activity of the mature protein.

The polypeptide can be a therapeutic polypeptide useful in human or veterinary therapy, i.e. a polypeptide that is physiologically active when introduced into the circulatory system of or otherwise administered to a human or an animal; a diagnostic polypeptide useful in diagnosis; or an industrial polypeptide useful for industrial purposes, such as in a manufacturing process where the polypeptide constitutes a functional ingredient or where the polypeptide is used for processing or other modification of raw ingredients during manufacturing.

The polypeptide can be of mammalian origin, e.g. of human, porcine, ovine, ursine, murine, rabbit, donkey, or bat origin, of microbial origin, e.g. of fungal, yeast or bacterial origin, or can be derived from other sources such as from venom, or from a leech, frog or mosquito. In the case of a therapeutic polypeptide, this is preferably of human origin, while an industrial polypeptide of interest is often of microbial origin.

Specific examples of groups of polypeptides that may be selected or expressed according to the invention include: an antibody or antibody fragment, a plasma protein, an erythrocyte or thrombocyte protein, a cytokine, a growth factor, a profibrinolytic protein, a binding protein, a protease inhibitor, an antigen, an enzyme, a ligand, a receptor, and a hormone. Of particular interest is a polypeptide that mediates its biological effect by binding to a cellular receptor when administered to a patient. In the case of an antibody, this can be a polyclonal or monoclonal antibody, and can be of any origin including human, rabbit and murine origin. Preferably, the antibody is a human or humanized monoclonal antibody. Specific antibodies and fragments thereof include those reactive with any of the therapeutic non-antibody proteins mentioned below.

### Antibodies

In one embodiment, the methods of the present invention can be applied to the selection and expression of antibodies to fulfill a wide variety of functions, determined largely by the selection of the target antigen or antigens.

As used herein, an "antibody" refers to a protein comprising one or more polypeptides substantially or partially encoded by immunoglobulin genes or fragments of immunoglobulin genes, e.g., a fragment containing one or more complementarity determining region (CDR). The recognized immunoglobulin genes include the kappa, lambda, alpha, gamma, delta, epsilon and mu constant region genes, as well as myriad immunoglobulin variable region genes. Light chains are typically classified e.g. as either kappa or lambda. Heavy chains are typically classified e.g. as gamma, mu, alpha, delta, or epsilon, which in turn define the immunoglobulin classes, IgG, IgM, IgA, IgD and IgE, respectively.

A typical immunoglobulin (antibody) structural unit comprises a tetramer. In nature, each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively.

Antibodies exist as intact immunoglobulins or as a number of well characterized fragments produced by digestion with various peptidases. Thus, for example, pepsin digests an antibody below the disulfide linkages in the hinge region to produce F(ab)'2 (fragment antigen binding) and Fc (fragment crystalizable, or fragment complement binding). F(ab)'2 is a dimer of Fab, which itself is a light chain joined to VH-CH1 by a disulfide bond. The F(ab)'2 may be reduced under mild conditions to break the disulfide linkage in the hinge region thereby converting the (Fab')2 dimer into an Fab' monomer. The Fab' monomer is essentially a Fab with part of the hinge region. The Fc portion of the antibody molecule corresponds largely to the constant region of the immunoglobulin heavy chain, and is responsible for the antibody's effector function (see Fundamental Immunology, 4th edition. W.E. Paul, ed., Raven Press, N.Y. (1998), for a more detailed description of antibody fragments). While various antibody fragments are defined in terms of the digestion of an intact antibody, one of skill will appreciate that such Fab' or Fc fragments may be synthesized de novo either chemically or by utilizing recombinant DNA methodology, peptide display, or the like. Thus, the term antibody, as used herein, also includes antibody fragments either produced by the modification of whole antibodies or synthesized de novo using recombinant DNA methodologies.

Antibodies also include single-armed composite monoclonal antibodies, single chain antibodies, including single chain Fv (scFv) antibodies in which a variable heavy and a variable light chain are joined together (directly or through a peptide linker) to form a continuous polypeptide, as well as diabodies, tribodies, and tetrabodies (Pack et al. 1995; Pack et al., 1993; Pack & Plueckthun, 1992). The antibodies are, e.g., polyclonal, monoclonal, chimeric, humanized, single chain, Fab fragments, fragments produced by an Fab expression library, or the like.

Using methods generally known in the art it is possible through the selection of appropriate target antigens to generate and evolve antibodies as treatment candidates for a number of human diseases; see e.g. WO 01/32712 for a detailed description of methods for antibody diversity generation as well as further information on particular antibodies. For example, diseases which result from a disregulation of the immune system, such as chronic inflammatory diseases, (e.g., lupus erythematosus, rheumatoid arthritis, and diabetes) and allergies, can respond favorably to antibodies which target components of the immune regulatory network, e.g., T cell and B cell surface determinants, superantigens, MHC class II, interferon gamma, alpha interferon, and leucointegrin. Similarly, optimized and humanized antibody reagents may be developed for the treatment of acute autoimmune disorders such as rhesus (rh) factor induced hydrops fetalis through the generation of improved recombinant anti-rh antibodies.

In addition, antibodies directed against other targets, such as markers isolated from vascular endothelium or activated epithelium, have potential in modulating the immune response. Similarly, antibodies to small molecule immunemodulators, such as nitrotyrosine, can play a role in regulating immune system disorders. Antibodies raised and optimized against allergens, for example, dust mite allergen, offer a potential therapeutic agent in the treatment of common allergies.

The methods of the invention may be used for selecting and/or expressing antibodies directed against Lymphocyte cell surface receptors and ligands (e.g., B7, CD80, CD86, CD28, and CTLA-4), Adhesion Molecules (e.g., LFA-1, Pgp-1, VLA-4, VCAM-1, ICAM-1, etc.), interleukins and their receptors (e.g., IL-2, IL-2R, etc.), and other cytokines (for example, interferon-gamma, tumor necrosis factor, alpha interferon, transforming growth factor-beta, etc., as well as e.g. any of the cytokines listed further below) and cytokine receptors, such as receptors for any of the cytokines listed further below. Also of interest are antibodies against Cluster of Differentiation (CD) antigens, for example: CD25, CD20, CD28, CD18, CD23, CD22, CD30, CD44, CD150 and their receptors, e.g., CD45R.

Antibodies for cancer immunotherapeutic agents are also candidates for selection and/or expression by the methods of the invention. Pan carcinoma markers as well as markers expressed on the surface of specific tumor types, e.g., bladder, breast, prostate, ovary, melanoma, glioma, lymphoma, and colorectal carcinoma, etc. can be isolated and used to generate monoclonal antibodies. Similarly, well known tumor growth factors, regulatory molecules, and markers including TNF-alpha, interferon gamma, ras, ErbB2, ErbB-3 R, adrenomedulin, Fas, EGF, EGF-R, rat neuT, Flk-1 receptor, vascular endothelial growth factor (VEGF), nsclc, pancarcinoma markers, carcinoembryonic antigen, (CEA), human chorionic gonadotrophin (HCG), and alphafetoprotein (AFP) are all suitable as antibody targets.

Neurological disorders such as Alzheimers disease can be addressed, for example, by developing optimized antibodies against beta-amyloid aggregates. Antibodies may also be developed for the treatment of such chronic degenerative disorders as Multiple Sclerosis. Antibodies also be optimized for use in the treatment of drug overdose, and toxicity, e.g., cocaine or antidepressants. Reagents useful for the diagnosis of neurological disorders may also be selected and/or expressed using the methods of the invention. For example, antibodies directed against neural components, such as HexosaminidaseA are valuable in the diagnosis of specific neurological disorders, e.g., Tay-Sachs disease.

Humanized antibodies optimized to bind proteins involved in lipid homeostasis, such as Cholesterol ester transfer protein (CETP), low density lipoprotein (LDL), and the atherosclerotic plaque marker, Z2D3, have potential utility in the diagnosis and treatment of hyperlipidemia and arteriosclerosis. Similarly, antibodies to human adipocytes have potential in the treatment of obesity. Antibodies directed against Type II phospholipase A2 are a possible reagent in the treatment of myocardial infarction, and antibodies against fibrin have potential in the treatment of clotting disorders.

Antibodies may also be used in the treatment of infectious diseases, including those caused by viral pathogens, e.g., Herpes Simplex Virus, Herpes zoster, Hepatitis A, B and C, Cytalomegalovirus, Respiratory syncitial virus, rabies, Human Papilloma Virus, Varicella zoster, B19 Parvovirus and viral agents causing the common cold, among others. Also of interest is coevolution of antibodies against HIV, including epitopes derived from envelope proteins, and including p17, gp120, gyp41, and p24. Antibodies can also be developed that are useful in the treatment of infectious diseases caused by bacterial agents, including enterococci, (e.g., *E. coli* verotoxin), *Bacillus psocyaneus* (flagellum), *Pneumocystis carinii, Pseudomonas aureuginosa, Staphylococcus epidermidis, Clostridium difficile, Cryptosporidium sp., Pseudomonas sp.,* and tetanus. Candidates for the treatment of fungal infections include ubiquitous heat shock proteins, e.g., the hsp90 of *Candida albicans,* which can be selected for high affinity binding, in spite of the limited antigenicity of the target antigen.

In addition to antibodies useful in the treatment or diagnosis of specific disorders as enumerated above, and as listed e.g. in Tables 1 and 2 of WO 01/32712, it will be clear that such various subsets of antibody classes as anti-idiotype antibodies, mimetic antibodies, anti-codon antibodies, bifunctional antibodies, diabodies, tribodies, tetrabodies, single chain antibodies, single-arm composite antibodies, monovalent antibodies, humanized antibodies, primatized antibodies, Trigger antibodies, antibody aggregates, and antibody-conjugates may all be selected and/or expressed by the methods of the invention. Antibody-conjugates include antibodies conjugated to protein moieties, (e.g., enzymes, nerve growth factor), chemotherapeutic or antiproliferative agents, (genistein, doxorubicin, calicheamicin, MX-DPTA, maytansine, mitomycin, etc.), radio-conjugates, (e.g., rhenium-186, rhenium-188, astatine-211, technetium-99, indium-111) and toxins, (e.g., PE38 and PE40 truncated *Pseudomonas* exotoxin, blocked ricin). Also included are antibodies conjugated to bioactive moieties such as vasoactive agents, and moieties which facilitate transport of the antibody across membranes or into the nucleus. Also contemplated are antibodies conjugated to non-biological particles such as gold, and magnetic nanoparticles (MNP, e.g., ranging from 10-50 nm in size).

Antibodies may e.g. be produced using naïve libraries of human antibodies (i.e. libraries obtained from subjects that have not been immunized with a particular target antigen) or from cells isolated from humans which are immunized with a target of interest (e.g., cells isolated from patients suffering from a disease such as HIV infection or any other condition which results in production of antibodies to a target). For example, any of the relevant targets can be used to screen naïve libraries of displayed antibodies (e.g., naïve human libraries). Alternatively, the targets can be used to elicit antibodies in animals such as mice or rabbits using standard methods. Antibody libraries comprising heavy and light chains may be created as separate mono-cistronic libraries of heavy chains or light chains or, using a bi-cistronic vector, as combined heavy and light chains within the same vector.

### Non-antibody polypeptides

- In the case of non-antibody therapeutic polypeptides, these can be selected from the following:
i) a plasma protein, e.g. a factor from the coagulation system, such as Factor VII, Factor VIII, Factor IX, Factor X, Factor XIII, thrombin, protein C, antithrombin III or heparin co-factor II, Tissue factor inhibitor (e.g. 1 or 2), endothelial cell surface protein C receptor, a factor from the fibrinolytic system such as pro-urokinase, urokinase, tissue plasminogen activator, plasminogen activator inhibitor 1 (PAI-1) or plasminogen activator inhibitor 2 (PAI-2), the Von Willebrand factor, or an α-1-proteinase inhibitor;
ii) an erythrocyte or thrombocyte protein, e.g. haemoglobin, thrombospondin or platelet factor 4;
iii) a cytokine, e.g. an interleukin such as IL-1 (e.g. IL-1α or IL-1β), IL-2, IL-3, IL-4, IL-5, IL-6, IL-9, IL-10, IL-11, IL-12, IL-13, IL-15, IL-16, IL-17, IL-18, IL-19, IL-20, IL-21, IL-22, IL-23, a cytokine-related polypeptide, such as IL-1Ra, an interferon such as interferon-α, interferon-β or interferon-y, a colony-stimulating factor such as GM-CSF or G-CSF, stem cell factor (SCF), a binding protein, a member of the tumor necrosis factor family (e.g. TNF-α, lymphotoxin-α, lymphotoxin-β, FasL, CD40L, CD30L, CD27L, Ox40L, 4-IBBL, RANKL, TRAIL, TWEAK, LIGHT, TRANCE, APRIL, THANK or TALL-1);
iv) a growth factor, e.g. platelet-derived growth factor (PDGF), transforming growth factor α (TGF-α), transforming growth factor β (TGF-β), epidermal growth factor (EGF), vascular endothelial growth factor (VEGF), somatotropin (growth hormone), a somatomedin such as insulin-like growth factor I (IGF-I) or insulin-like growth factor II (IGF-II), erythropoietin (EPO), thrombopoietin (TPO) or angiopoietin;
v) a profibrinolytic protein, e.g. staphylokinase or streptokinase;
vi) a protease inhibitor, e.g. aprotinin or CI-2A;
vii) an enzyme, e.g. superoxide dismutase, catalase, uricase, bilirubin oxidase, trypsin, papain, asparaginase, arginase, arginine deiminase, adenosin deaminase, ribonuclease, alkaline phosphatase, β-glucuronidase, purine nucleoside phosphorylase or batroxobin;
viii) an opioid, e.g. endorphins, enkephalins or non-natural opioids;
ix) a hormone or neuropeptide, e.g. insulin, calcitonin, glucagons, adrenocorticotropic hormone (ACTH), somatostatin, gastrins, cholecystokinins, parathyroid hormone (PTH), luteinizing hormone (LH), follicle-stimulating hormone (FSH), gonadotropin-releasing hormone, chorionic gonadotropin, corticotropin-releasing factor, vasopressin, oxytocin, antidiuretic hormones, thyroid-stimulating hormone, thyrotropin-releasing hormone, relaxin, glucagon-like peptide 1 (GLP-1), glucagon-like peptide 2 (GLP-2), prolactin, neuropeptide Y, peptide YY, pancreatic polypeptide, leptin, orexin, CART (cocaine and amphetamine regulated transcript), a CART-related peptide, melanocortins (melanocyte-stimulating hormones), melanin-concentrating hormone, natriuretic peptides, adrenomedullin, endothelin, exendin, secretin, amylin (IAPP; islet amyloid polypeptide precursor), vasoactive intestinal peptide (VIP), pituitary adenylate cyclase activating polypeptide (PACAP), agouti and agouti-related peptides or somatotropin-releasing hormones; or
x) another type of therapeutic protein or peptide such as thymosin, bombesin, bombesin-like peptides, heparin-binding protein, soluble CD4, pigmentary hormones, hypothalamic releasing factor, malanotonins, phospholipase activating protein, a detoxifying enzyme such as acyloxyacyl hydrolase, or an antimicrobial peptide.

In the case of an industrial polypeptide, this is typically an enzyme, in particular a microbial enzyme used in products or in the manufacture of products such as detergents, household articles, personal care products, agrochemicals, textile, food products, in particular bakery products, feed products, or in industrial processes such as hard surface cleaning. The industrial polypeptide is normally not intended for internal administration to humans or animals. Specific examples include hydrolases, such as proteases, lipases or cutinases, oxidoreductases, such as laccase and peroxidase, transferases such as transglutaminases, isomerases, such as protein disulphide isomerase and glucose isomerase, cell wall degrading enzymes such as cellulases, xylanases, pectinases, mannanases, etc., amylolytic enzymes such as endoamylases, e.g. alpha-amylases, or exo-amylases, e.g. beta-amylases or amyloglucosidases, etc.

### Regulated Readthnough approaches

The stop codon, also known as a chain termination codon, used in the method of the invention may be any one or more of three codons, UAA, UAG and UGA, that signal termination of synthesis of a protein. Although expression cassettes for use in methods of the invention will normally comprise only a single stop codon upstream of the coding sequence for the cell membrane anchoring peptide, reporter peptide, epitope tag or antibiotic resistance gene, it is also possible to use a series of two or more stop codons, e.g. two or three stop codons, which may the same or different. As will be described in more detail below, there is generally a very low level of stop codon readthrough even in the absence of a chain termination agent. Depending on factors such as the natural level of background readthrough for a given stop codon in a given construct and the aim of a particular selection method according to the invention, it may in some cases be desirable to use more than one stop codon in order to further reduce background readthrough. Similarly, readthrough levels with and without a termination suppression agent may also be adjusted by selection of a suitable stop codon when only a single stop codon is used. As is described elsewhere herein, the tetranucleotide context of the stop codon, i.e. the trinucleotide stop codon itself as well as the nucleotide immediately downstream of the stop codon, also has an influence on readthrough levels.

In addition to the possible use of multiple stop codons following the gene of interest, it will normally be advantageous to use multiple stop codons downstream of the sequence encoding the cell membrane anchoring peptide, reporter peptide, epitope tag or antibiotic resistance gene. The use of multiple stop codons in this position, e.g. up to about ten stop codons, such up to about six or eight stop codons, such as about two, three, four or five stop codons, will ensure efficient termination of translation even in the presence of the termination suppression agent.

The term "cell membrane anchoring peptide" refers to a peptide or protein that serves to anchor the polypeptide of interest to a cell membrane, either directly or indirectly. Indirect anchoring refers to situations in which the cell membrane anchoring peptide is not anchored in the cell membrane itself, but rather is indirectly attached to the lipid membrane bilayer as in the case of a GPI (glycosyl-phosphatidylinositol) anchor. Direct anchoring refers to situations in which the cell membrane anchoring peptide is directly embedded in and anchored to the lipid bilayer of the membrane. Polypeptides which are anchored to the cell membrane via an anchoring peptide will be displayed at the surface of the cell and can thus be identified, e.g. by FACS, or alternatively by other methods such as other fluorescence-based methods, ELISA or other affinity-based methods, or radioactivity-based methods. A preferred method is FACS, however, due to its high-throughput screening capacity that allows rapid and efficient screening of very large cell populations.

For purposes of screening using e.g. FACS, the cell membrane targeting signal is normally positioned at the COOH end of the protein fusion (downstream of the stop codon except where otherwise indicated herein). Additionally, it is important that the soluble part of the protein (i.e. the polypeptide of interest) is displayed on the right side of the membrane (the extracellular side) for subsequent antibody/ligand interaction during FACS. A preferred anchoring peptide is the GPI anchor.

Many different types of proteins such as enzymes, receptors, protozoal antigens and mammalian antigens in a variety of eukaryotic organisms have been found to be bound to the plasma membrane by GPI anchors (Ikezawa, 2002). Numerous GPI anchor sequences are known in the art, and the use of such GPI anchors for protein expression is described e.g. in WO 89/01041, WO 90/12099 and WO 95/22614. An example of a suitable GPI anchor for purposes of the present invention is the human placental alkaline phosphatase (HPAP) GPI anchor with the sequence LEPTYCDLAPPAGTTDAAHPGRSVVP-ALLPLLAGTLLLLETATAP (SEQ ID NO:7) (which is a slightly modified version of the sequence described by Millan, 1986).

An example of another anchoring domain suitable for use in the methods of the invention is the C-terminal transmembrane anchoring domain of platelet derived growth factor receptor (PDGFR) with the sequence AVGQDTQEVIVVPHSLPFKVVVISAIL-ALVVLTIISLIILIMLWQKKPR (SEQ ID NO:8) (Kawagishi et al., 1995).

In a preferred embodiment, fusion proteins comprising a polypeptide of interest fused to a cell membrane anchoring peptide are sorted using Fluorescence-Activated Cell Sorting (FACS). In the context of the present invention, FACS sorting of membrane-bound fusion proteins is particularly advantageous, since it allows rapid screening of large numbers of cells to identify those in which the termination suppression agent has resulted in translational readthrough, as only these cells will express the polypeptide of interest at the cell surface in the form of a fusion protein comprising the polypeptide of interest and the cell membrane anchoring peptide. Once these cells have been identified by FACS, they can then be cultured in the absence of the termination suppression agent to result in production of the polypeptide of interest as a soluble polypeptide without the anchoring peptide. Surprisingly, the inventor has found that there is a positive and statistically significant correlation between fluorescence, as determined by FACS, and soluble protein activity levels. Thus, FACS sorting can be used in the method of the invention not only for qualitative analysis to identify cells expressing a protein of interest, but can actually be used quantitatively to identify cells that express high levels of a given protein. It has further been found that the methods of the invention are advantageous for evaluating heterogeneity of protein expression, i.e. for identifying and selecting cells or cell clones that exhibit both a desired level and a desired uniformity of protein expression.

The term "reporter peptide" refers to a peptide or protein that may readily be assayed by suitable means, thereby allowing easy detection of fusion proteins comprising a polypeptide of interest and the reporter peptide. A number of different reporter peptides are well-known in the art and include green fluorescent protein (GFP), luciferase, β-galactosidase, β-glucuronidase and chloramphenicol acetyltransferase (CAT).

An "epitope tag" refers to a short amino acid sequence that serves as an antibody recognition site (epitope), allowing detection of a fusion protein comprising the polypeptide of interest and the epitope tag e.g. by means of fluorescently labeled antibodies that bind to the tag. Numerous epitope tags are known in the art, and products for detecting epitope tags, e.g. antibodies such as fluorescently labeled antibodies, are commercially available. Examples of epitope tags include V5 (GKPIPNPLLGLDST) (SEQ ID NO:9), His₆ (HHHHHH) (SEQ ID NO:10), FLAG™ (DYKDDDDKG) (SEQ ID NO:11), HA (YPYDVPDYA) (SEQ ID NO:12), c-Myc (EQKLISEEDL) (SEQ ID NO:13), VSV-G (YTDIEMNRLGK) (SEQ ID NO:14), and HSV (QPELAPEDPED) (SEQ ID NO:15).

The expression cassette may if desired include sequences that code for two or more of a cell membrane anchoring peptide, a reporter peptide and an epitope tag. For example, it may comprise a cell membrane anchoring peptide together with either a reporter peptide or an epitope tag, thus allowing the polypeptide of interest to be displayed at the cell surface in the form of a membrane-anchored fusion protein which may be screened or selected not only by FACS but also via the reporter peptide or epitope tag. In this case, the stop codon will be located downstream of the coding sequence for the polypeptide of interest but upstream of the coding sequences for the anchoring peptide and the reporter peptide or epitope tag.

Alternatively, in particular for proteins which in their native form are targeted to the plasma membrane, e.g. hormone receptors, the stop codon may be located downstream of the sequence encoding the cell membrane anchoring peptide but upstream of the sequence encoding the reporter peptide. In this case, expression in the presence of an aminoglycoside results in a non-native fusion protein that can be sorted or selected e.g. by FACS or affinity chromatography on the basis of the reporter peptide, while expression in the absence of an aminoglycoside results in a "native-type" membrane-bound protein comprising the polypeptide of interest. The term "native-type" in this context refers to the fact that the fusion protein comprises a non-tagged form of the polypeptide of interest (where the polypeptide of interest may be a mutagenized form of a "native" polypeptide) that is naturally targeted to the cell membrane.

In a further alternative embodiment, a polynucleotide encoding an epitope tag or reporter peptide, in particular an epitope tag, may be included before the stop codon, with a polynucleotide encoding an anchoring peptide after the stop codon, to generate the following construct: gene of interest-tag-STOP-anchor. In this case, the method is suitable for selecting cell lines producing high levels of soluble tagged protein by FACS. The tag may e.g. be a His tag, a V5 epitope tag, or any of the other tags or reporter peptides listed above.

The "termination suppression agent" is a chemical agent which is able to suppress translational termination resulting from the presence of a stop codon. In particular, the termination suppression agent is an antibiotic belonging to the aminoglycoside group. As explained above, aminoglycoside antibiotics are known for their ability to allow insertion of alternative amino acids at the site of a stop codon, thereby resulting in "readthrough" of a stop codon that otherwise normally would result in chain termination. Aminoglycoside antibiotics include G-418 (Geneticin®), gentamicin (gentamycin), paromomycin, hygromycin, amikacin, kanamycin, neomycin, netilmicin, paromomycin, streptomycin and tobramycin.

It will be understood by persons skilled in the art that even in the absence of a termination suppression agent, there will generally be a small level of background stop codon readthrough. The degree of background readthrough varies somewhat depending on the particular stop codon, including the tetranucleotide context, and readthrough may also vary among different aminoglycoside antibiotics. Similarly, for a given stop codon and termination suppression agent, the degree of translational readthrough may be adjusted by varying the concentration of the termination suppression agent. These differences in background readthrough and in translational readthrough obtained with different stop codons and termination suppression agents may be used advantageously in the context of the present invention in order to select combinations of stop codons/tetranucleotides and aminoglycosides that provide the desired result. For example, the background readthrough of the UAA stop codon is lower than for the UGA stop codon, while higher translational readthrough rates are obtainable using, e.g., G-418 with a UGA stop codon than with a UAA stop codon.

In one experiment with G-418, for example, the present inventor obtained up to about 25% FACS-positive cells for the UGA stop codon (UGAC tetranucleotide), but only up to about 10% FACS-positive cells for the UAA stop codon (UAAC tetranucleotide). The background levels of FACS-positive cells in the absence of G-418 in this case were about 13% and 0.5%, respectively, for UGAC and UAAC. By way of example, the UAA stop strategy may therefore be used for selecting high-expressing clones by FACS prior to production of soluble protein in the absence of an aminoglycoside, since the UAA construct has almost no background readthrough. Conversely, the UGA stop strategy may be a good alternative when maximum levels of readthrough are wanted and background readthrough is not a concern, e.g. for functional library screening.

As indicated above, one aspect of this disclosure relates to methods for screening or selecting cell clones expressing a high level of a polypeptide of interest, but where use of an aminoglycoside is unnecessary. Surprisingly, it has been found that for the purpose of selecting cell clones that express a desired level of a polypeptide of interest, efficient selection may be performed without an aminoglycoside based on a low yet detectable level of background readthrough in high expressing cells, resulting in a fusion protein comprising the polypeptide fused to a cell membrane anchoring peptide that allows display of the fusion protein at the cell surface. This approach of non-aminoglycoside-based selection of cell clones having high and uniform expression levels can, for example, be used subsequent to selection of cells expressing a polypeptide with desired properties from a library using any of the methods described herein.

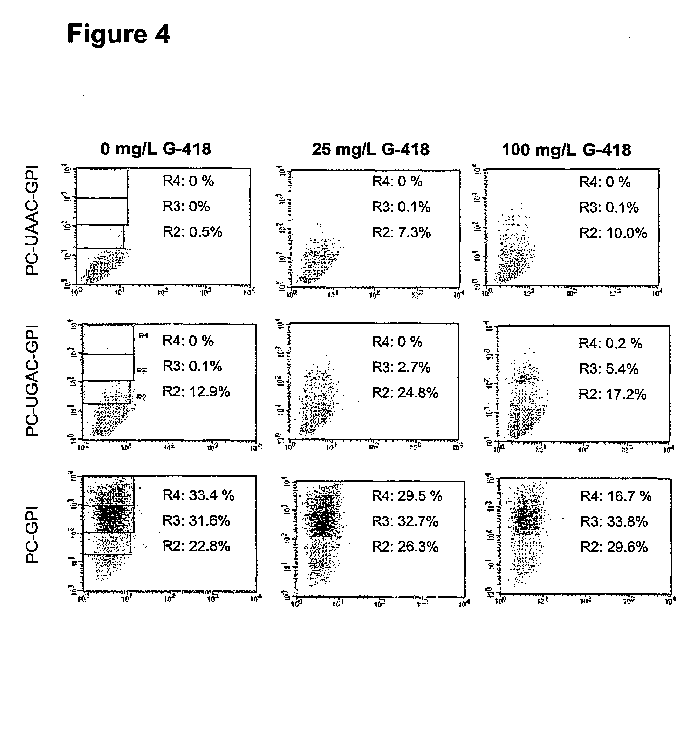

As explained elsewhere herein, the inventor has found that readthrough levels in the presence of an aminoglycoside are generally correlated with protein expression levels, thus allowing efficient selection of high expressing clones, but it has also been found that this same approach can be used even without aminoglycoside-mediated readthrough. This is illustrated in Figure 4 (see Example 2), which shows that even without the aminoglycoside antibiotic G-418, there is still a significant level of background translational read through, i.e. a 12.9% readthrough level in gate R2 for the PC-UGAC-GPI construct.

In the context of the present invention the term "soluble protein" or "soluble polypeptide" refers to the polypeptide of interest when expressed in soluble form without being fused to a cell membrane anchoring peptide. The soluble protein is thus generally obtained by expression in the absence of a termination suppression agent, whereby the at least one stop codon downstream of the first polynucleotide effectively results in chain termination so that the polypeptide of interest is not membrane-bound. If desired, however, the soluble polypeptide may be expressed together with a reporter peptide or epitope tag, the coding sequence for the reporter peptide or tag in this case being located upstream of the stop codon(s).

As indicated above, there are disclosed herein methods suitable for use as alternatives to conventional antibiotic-based selection of cells transformed with a gene of interest. This allows for efficient selection of cells that have been transformed with the gene of interest, but has the advantage compared to antibiotic resistance-based selection methods of also allowing the resulting selected cells to be used for production of the polypeptide of interest without undesired expression of an antibiotic resistance gene. In a preferred embodiment of this aspect of the disclosure, no antibiotic resistance gene is present in the expression cassette comprising the gene of interest, the stop codon and the cell targeting peptide, i.e. in this case selection of cells expressing the polypeptide of interest is not based on antibiotic resistance. Instead, selection is related to the presence of the cell targeting peptide.

As used herein, a "cell targeting peptide" is a peptide or protein that targets the polypeptide of interest to the cell in which it is produced, i.e. to either the interior of the cell or linked to the exterior of the cell. Examples of suitable cell targeting peptides include membrane targeting peptides such as the GPI anchor, e.g. for cases where antibodies directed against the polypeptide of interest-cell targeting peptide fusion are to be used during FACS sorting, as well as any peptide that targets the fusion to cell compartments in the interior of the cell. Cell targeting peptides that may be used for intracellular targeting include e.g. a nuclear localization signal (NLS), a signal targeting the polypeptide to other sub-cellular compartments (e.g. the cytoplasm, mitochondria or endoplasmic reticulum), and cellular structures such as microtubules. For intracellular targeting, it will be understood that at least one of the proteins belonging to the polypeptide of interest-cell targeting peptide fusion has intrinsic biochemical properties allowing its detection within the cell, for example by fluorescence.

In another embodiment of this aspect of the disclosure, selection of cells expressing the polypeptide of interest may be performed using a conventional antibiotic resistance technique, but where the presence of one or more stop codons downstream of the gene of interest and upstream of the antibiotic resistance gene ensures that the antibiotic resistance gene is not expressed under normal production conditions in the absence of an aminoglycoside antibiotic. Selection using this embodiment of the disclosure will normally employ two different antibiotics in the selection medium, i.e. an aminoglycoside antibiotic that results in translational readthrough and expression of the antibiotic resistance gene, and a non-aminoglycoside antibiotic used for the actual selection. Cells transformed with the expression cassette containing the gene of interest will thus express the antibiotic resistance gene, which provides resistance to the non-aminoglycoside antibiotic, but only in the presence of an aminoglycoside antibiotic that allows translational readthrough of the stop codon(s). Any non-aminoglycoside antibiotic may be used as the antibiotic for selection in this embodiment, e.g. ampicillin, bleomycin, phleomycin, spectinomycin, blasticidin, puromycin, zeocin, etc.

In any of the methods described herein, it may be advantageous to culture the transformed cells in the presence of a butyrate salt, e.g. sodium butyrate, in order to increase expression levels of the polypeptide of interest (see e.g. Gorman et al., 1983). Even in the presence of an aminoglycoside, stop codon readthrough levels may still be relatively low, and it will therefore often be desirable to increase expression levels to be able to more easily detect the polypeptide of interest. This may particularly be the case for screening of expression libraries based on stop codon readthrough resulting in expression of the polypeptide of interest fused to a cell membrane anchoring peptide. The butyrate salt will typically be used in a concentration of about 1-10 mM, depending on the cell type. For CHO cell expression, for example, a suitable concentration is about 1-2 mM (Hunt et al., 2002).

The present invention is applicable to any type of host cell from organisms in which translational stop codon readthrough is promoted in the presence of aminoglycosides, in particular eukaryotic cells such as mammalian cells or other animal cells, filamentous fungal cells, yeast cells, insect cells, and transgenic plants and animals. Examples of suitable mammalian host cells include Chinese hamster ovary (CHO) cell lines, (e.g. CHO-K1; ATCC CCL-61), Green Monkey cell lines (COS) (e.g. COS 1 (ATCC CRL-1650), COS 7 (ATCC CRL-1651)); mouse cells (e.g. NS/O), Baby Hamster Kidney (BHK) cell lines (e.g. ATCC CRL-1632 or ATCC CCL-10), and human cells (e.g. HEK 293 (ATCC CRL-1573)).

Examples of suitable filamentous fungal host cells include strains of *Aspergillus,* e.g. *A. oryzae, A. niger,* or *A. nidulans, Fusarium* and *Trichoderma.* Examples of suitable yeast host cells include strains of *Saccharomyces,* e.g. *S*. *cerevisiae, Schizosaccharomyces, Klyveromyces, Pichia,* such as *P. pastoris* or *P. methanolica, Hansenula,* such as *H. polymorpha,* and *Yarrowia.* Examples of suitable insect host cells include a *Lepidoptora* cell line, such as *Spodoptera frugiperda* (Sf9 or Sf21) or *Trichoplusioa ni* cells (High Five) (US 5,077,214).

Preferably, the cells used in the methods of the invention are selected from mammalian cells and yeast cells.

Persons skilled in the art will be capable of selecting suitable vectors, expression control sequences and hosts for performing the methods of the invention. For example, in selecting a vector, the host must be considered because the vector must be able to replicate in it or be able to integrate into the chromosome. The vector's copy number, the ability to control that copy number, and the expression of any other proteins encoded by the vector, such as antibiotic markers, should also be considered. The vector may be any vector known in the art, in particular a plasmid or viral vector. For library screening methods, an example of a suitable vector is a retroviral vector. Retroviral vectors are advantageous for this purpose in that they allow easy control of the copy number (e.g. to provide a single vector per cell), and they also allow high library titers due to a high infection efficiency. For production purposes, in particular for the production of therapeutic proteins, it is preferred to use a non-retroviral vector in order to eliminate a possible risk of development of infectious recombinant retrovirus. Both retroviral and non-retroviral vectors are commercially available.

In selecting an expression control sequence, a variety of factors should also be considered. These include, for example, the relative strength of the sequence, its controllability, and its compatibility with the nucleotide sequence encoding the polypeptide, particularly as regards potential secondary structures. Hosts should be selected by consideration of their compatibility with the chosen vector, possible toxicity of the product coded for by the nucleotide sequence, their secretion characteristics, their ability to fold the polypeptide correctly, their fermentation or culture requirements, and the ease of purification of the products coded for by the nucleotide sequence.

The term "control sequences" is defined herein to include all components which are necessary or advantageous for the expression of polypeptides according to the invention. Each control sequence may be native or foreign to the nucleic acid sequence encoding the polypeptide. Such control sequences include, but are not limited to, a leader sequence, polyadenylation sequence, propeptide sequence, promoter, enhancer or upstream activating sequence, signal peptide sequence, and transcription terminator. The control sequences will generally include at least a promoter and a signal peptide.

Examples of suitable control sequences for directing transcription in mammalian cells include the early and late promoters of SV40 and adenovirus, e.g. the adenovirus 2 major late promoter, the MT-1 (metallothionein gene) promoter, the human cytomegalovirus immediate-early gene promoter (CMV), the human elongation factor 1α (EF-1α) promoter, the Drosophila minimal heat shock protein 70 promoter, the Rous Sarcoma Virus (RSV) promoter, the human ubiquitin C (UbC) promoter, the human growth hormone terminator, SV40 or adenovirus Elb region polyadenylation signals and the Kozak consensus sequence (Kozak, 1987).

In order to improve expression in mammalian cells a synthetic intron may be inserted in the 5' untranslated region of the nucleotide sequence encoding the polypeptide. An example of a synthetic intron is the synthetic intron from the plasmid pCI-Neo (available from Promega Corporation, WI, USA).

Examples of suitable control sequences for directing transcription in insect cells include the polyhedrin promoter, the P10 promoter, the *Autographa californica* polyhedrosis virus basic protein promoter, the baculovirus immediate early gene 1 promoter and the baculovirus 39K delayed-early gene promoter, and the SV40 polyadenylation sequence.

Examples of suitable control sequences for use in yeast host cells include the promoters of the yeast α-mating system, the yeast triose phosphate isomerase (TPI) promoter, promoters from yeast glycolytic genes or alcohol dehydogenase genes, the ADH2-4c promoter and the inducible GAL promoter.

Examples of suitable control sequences for use in filamentous fungal host cells include the ADH3 promoter and terminator, a promoter derived from the genes encoding *Aspergillus oryzae* TAKA amylase triose phosphate isomerase or alkaline protease, an A. *niger* α-amylase, *A. niger* or *A. nidulans* glucoamylase, *A. nidulans* acetamidase, *Rhizomucor miehei* aspartic proteinase or lipase, the TPI1 terminator and the ADH3 terminator.

For purposes of the present invention, a signal peptide will generally be present to obtain expression of the polypeptide of interest either in membrane-anchored form or in secreted, soluble form. Such signal peptide should be one recognized by the cell chosen for expression of the polypeptide. The signal peptide may be homologous (e.g. be that normally associated with the polypeptide in question) or heterologous (i.e. originating from another source) to the polypeptide or may be homologous or heterologous to the host cell, i.e. be a signal peptide normally expressed from the host cell or one which is not normally expressed from the host cell.

In production methods of the present invention, cells are cultivated in a nutrient medium suitable for production of the polypeptide in question using methods known in the art. For example, the cells may be cultivated by shake flask cultivation, small-scale or large-scale fermentation (including continuous, batch, fed-batch, or solid state fermentations) in laboratory or industrial fermenters performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). When the polypeptide is secreted into the nutrient medium; the polypeptide can be recovered directly from the medium.

As explained elsewhere herein, selection or screening of polypeptides according to the methods of the invention may be performed by any suitable means, e.g. by FACS in the case of membrane bound polypeptides or by suitable detection of a reporter peptide or epitope tag.

Polypeptides produced in accordance with the invention may be recovered by methods known in the art. For example, the polypeptide may be recovered from the nutrient medium by conventional procedures including, but not limited to, centrifugation, filtration, ultra-filtration, extraction or precipitation. Purification may be performed by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation) or extraction (see, e.g., Protein Purification (2nd Edition), Janson and Ryden, editors, Wiley, New York, 1998).

The present invention also provides kits including the expression cassettes, expression vectors, cells and methods of the invention. Kits of the invention optionally comprise at least one of the following of the invention: (1) at least one kit component comprising an expression cassette as described herein suitable for performing a method of the invention; a cell or expression cassette comprising such an expression cassette; an aminoglycoside antibiotic; or a composition comprising at least one such component; (2) instructions for practicing any method described herein, instructions for using any component identified in (1) or any composition of any such component; (3) a container for holding said at least one such component or composition, and (4) packaging materials. Typically, the kit will comprise at least one component of (1) together with instructions for use and a container and/or packaging materials. The individual components of the kit may be packaged together or separately.

There is also disclosed herein the use of any apparatus, component, composition, or kit described above and herein, for the practice of any method or assay described herein, and/or for the use of any apparatus, component, composition, or kit to practice any assay or method described herein.

The invention is further illustrated by the following non-limiting examples.

### EXAMPLES

### Example 1: Construction of pLenti6-PC-GPI, pLenti6-PC-UAAC-GPI and pLenti6-PC-UGAC-GPI

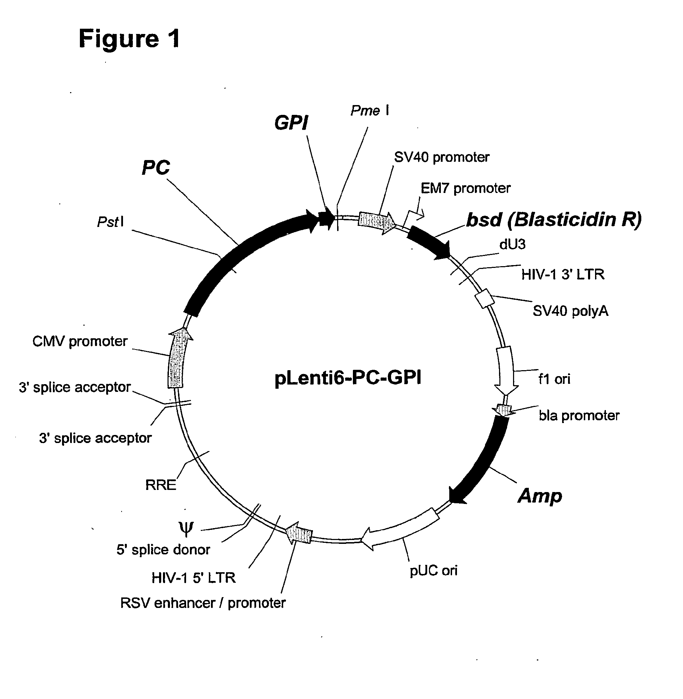

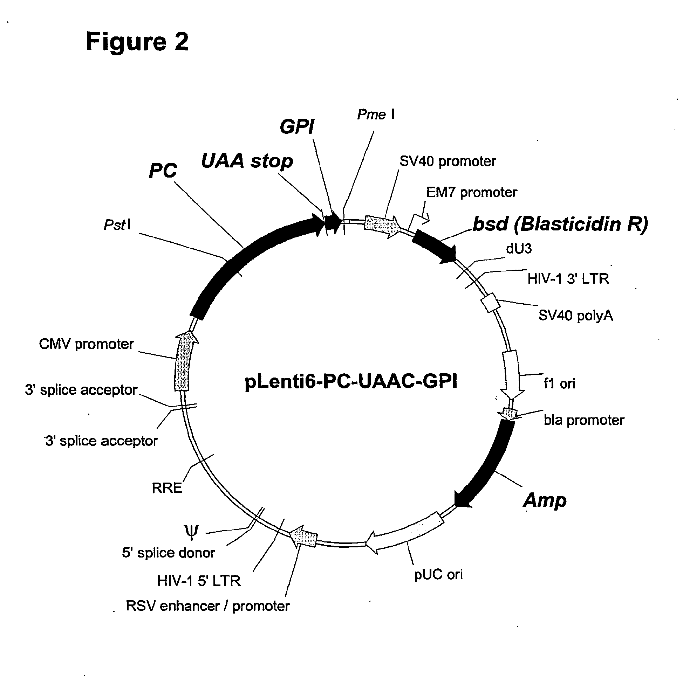

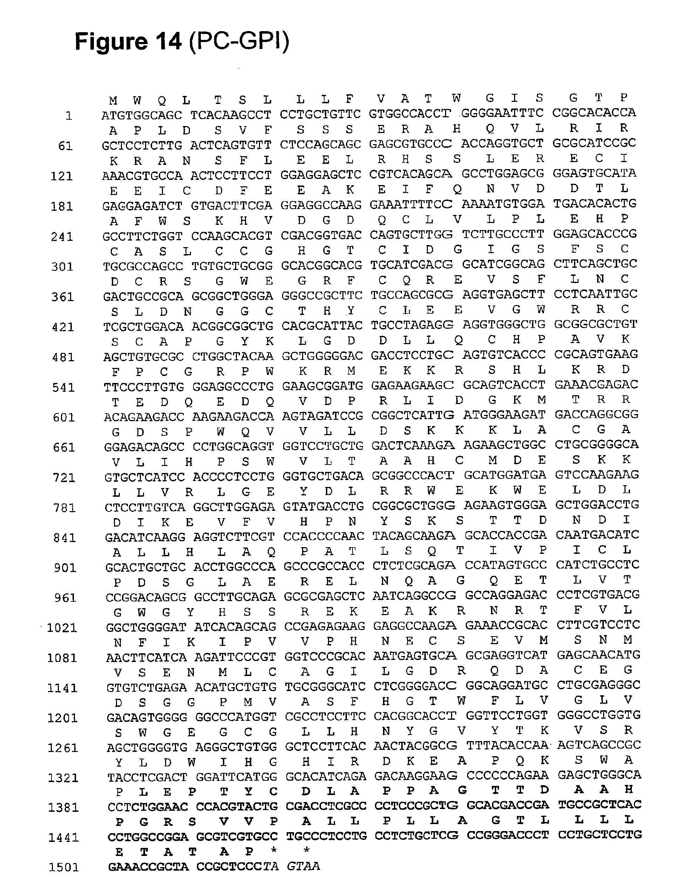

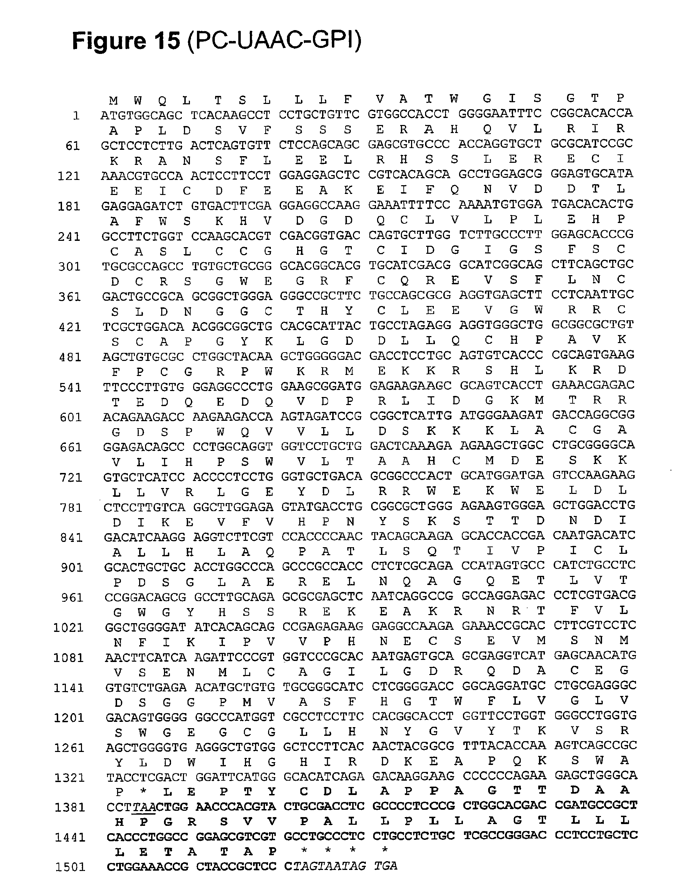

To construct the pLenti6-PC-GPI vector (Figure 1), a translational fusion between sequences encoding human Protein C (PC) and the GPI anchor was amplified by PCR (polymerase chain reaction) using the primers TBO017 (5'-CACCATGTGGCAGCTCACAAGCC-3') (SEQ ID NO:16) and TBO014 (5'-AGAAGGCACAGTCGAGGCTGATC) (SEQ ID NO:17). A vector containing a fusion between the PC sequence and the GPI sequence (Figure 14) was used as a template. The resulting PCR product was cloned into the vector pLenti6/V5-D-TOPO (Invitrogen) using the procedure recommended by the manufacturer. For constructing the pLenti6-UAAC-GPI vector, the pLenti6-PC-GPI vector served as a template for two independent PCR reactions:
the coding sequence of PC was amplified using the primers TBO077 (5'-CGGTGACCAGTGCTTGGTCTTGC-3') (SEQ ID NO:18) and TBO103 (5'-CAGTACGTGGGTTCCAGTTAAGGTGCCCAGCTCTTCTGGGGGGCTTCC-3') (SEQ ID NO:19). In a second PCR reaction, the GPI anchoring sequence was amplified using the primers TBO102 (5'- CCAGAAGAGCTGGGCACC-TTAACTGGAACCCACGTACTGCGACCTCGC-3') (SEQ ID NO:20) and TBO104 (5'-ATCAGCGGTTTAAACTTTCACTATTACTAGGGAGCGGTAGCGGTTTCC-3') (SEQ ID NO:21). The two resulting PCR products served as templates in a fusion PCR procedure using the primers TBO077 and TBO104. The resulting PCR fragment was cleaved using the restriction endonucleases PstI and PmeI and ligated into the vector *pLenti6-PC-GPI* (Figure 1) at the corresponding endonuclease sites, *giving pLenti6-PC-UAAC-GPI* (Figure 2). This expression vector harbors the PC sequence translationally fused to the GPI anchor sequence and a UAA stop codon between the coding sequences of the PC and the GPI anchor (Figure 15). Additionally, four stop codons follow the GPI anchor to efficiently terminate the translation even in the presence of aminoglycosides.

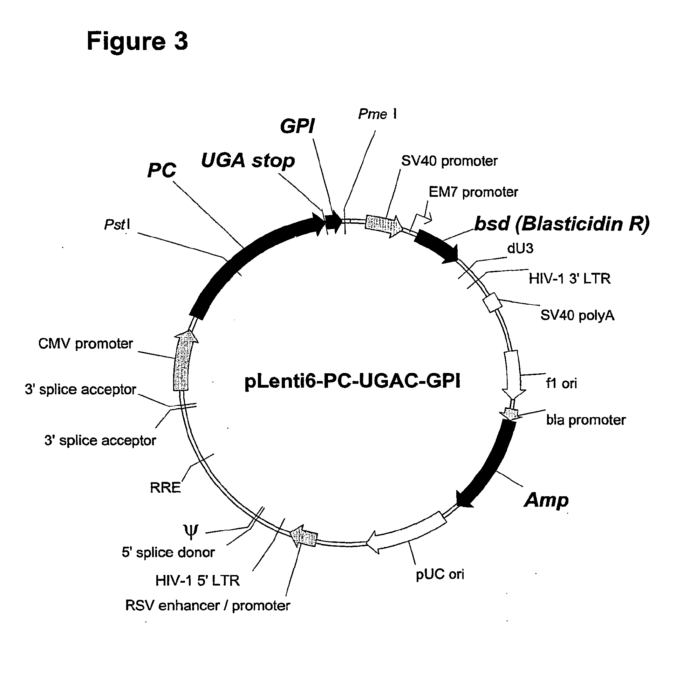

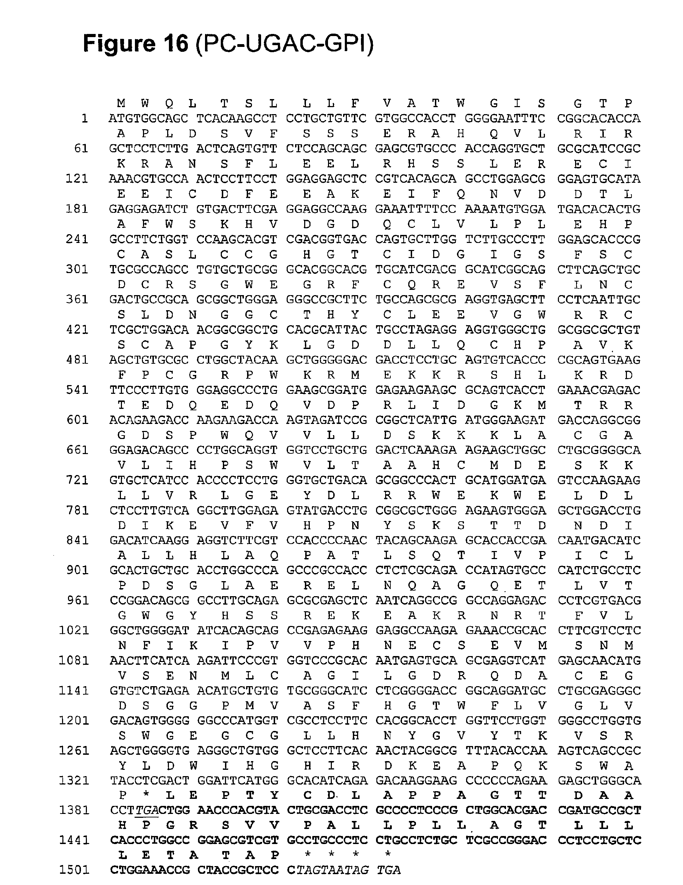

For constructing the *pLenti6-UGAC-GPI* vector, the pLenti6-PC-GPI vector served as template for two independent PCR reactions: the coding sequence of the human PC was amplified using the primers TBO077 and TBO108 (5'- CAGTACGTGGGTTCCAGTC-AAGGTGCCCAGCTCTTCTGGGGGGCTTCC-3') (SEQ ID NO:22). In a second PCR reaction, the GPI anchoring sequence was amplified using the primers TBO107 (5'-CCAGAAGAGCTGGGCACCTTGACTGGAACCCACGTACTGCGACCTCGC-3') (SEQ ID NO:23) and TBO104. The two resulting PCR products served as templates in a fusion PCR procedure using the primers TBO077 and TBO104 The resulting PCR fragment was cleaved using the restriction endonucleases PstI and PmeI and ligated into the vector *pLenti6-PC-GPI* (Figure 1) at the corresponding endonuclease sites, giving *pLenti6-PC-UGAC-GPI* (Figure 3). This expression vector harbors the protein C sequence translationally fused to the GPI anchor sequence and a UGA stop codon between the coding sequences of the PC and the GPI anchor (Figure 16). Additionally, four stop codons follow the GPI anchor to efficiently terminate the translation even in the presence of aminoglycosides.

Using the same general approach, similar vectors may be prepared using the coding sequence for any desired polypeptide instead of the coding sequence for human protein C.

### Example 2: Aminoglycoside-induced in vivo suppression of termination

To demonstrate that a recombinant gene expression vector as disclosed herein can be used for aminoglycoside-induced *in vivo* suppression of termination, the retroviral vectors *pLenti6-PC-UAAC-GPI* and *pLenti6-PC-UGAC-GPI* were used to transfect HEK293FT cells (Invitrogen) using the Lipofectamine™ 2000 (Invitrogen) transfection reagent. As a control, the retroviral vector *pLenti6-PC-GPI* was used to transfect HEK293FT cells and produce retrovirus particles. After 48 hours, supernatants containing retroviral particles were harvested, filter-sterilized to remove cell debris, and subsequently used to infect CHO-K1 cells. CHO-K1 cells were selected for resistance to the Blasticidin antibiotic at the concentration of 5 mg/l for 10 days. The resulting pools of Blasticidin-resistant cells were transferred into 6 culture flasks for each cell pool and grown to 25% confluency. To induce translational readthrough, the antibiotic G-418 was added to the culture flasks at final concentrations ranging from 12.5 mg/l to 100 mg/l and flasks were incubated for another 48 hours at 37°C. Cells were detached from the flasks by trypsinization and were incubated with mouse anti-human PC monoclonal antibodies. Cells were subsequently washed and incubated with a secondary antibody (rabbit anti-mouse IgG, Phycoerythrin-labeled (DAKO R0439)). Labeled retroviral cell lines were analyzed by FACS for membrane-anchored recombinant PC using a FACScalibur™ (Becton Dickinson) instrument with an excitation wave length of 488 nm and an emission filter of 585 nm.

The results shown in Figure 4 illustrate that both cell lines expressing the PC-UAAC-GPI and PC-UGAC-GPI reporters display PC at the cell surface in the presence of aminoglycoside. Moreover, the amount of recombinant protein that is detected (Y axis) is proportional to the G-418 concentration. Furthermore, the amount of recombinant membrane-anchored PC is more abundant for the PC-UGAC-GPI construct than for the PC-UAAC-GPI construct, and this applies to all aminoglycoside concentrations that were assessed. This result is in accordance with aminoglycoside-mediated translational readthrough performed in an *in vitro* model (Manuvakhova et al., 2000). In contrast to the retroviral cell lines expressing the PC-UAAC-GPI and PC-UGAC-GPI reporters, the retroviral cell line expressing the PC-GPI reporter does not exhibit an increased amount of displayed recombinant protein in the presence of aminoglycoside. This result was expected considering that the expression cassette does not harbor a stop codon between the PC and GPI sequences. This result also confirms that both the UAAC and the UGAC tetranucleotides can be successfully used to modulate aminoglycoside-mediated *in vivo* translational readthrough.

### Example 3. Efficient selection of cell clones expressing high levels of recombinant protein C by FACS

Until now, selection of clones expressing high levels of recombinant soluble protein has been a labor-intensive task that typically limits the number of clones that can be analyzed to a few hundred. Furthermore, because the expression of the selectable marker gene does not directly correlate with the expression levels of the gene of interest, most of the clones do not express satisfactory recombinant protein levels. FACS-based sorting of cells offers a high-throughput screening capacity that allows the daily analysis/sorting of cell populations greater than 1,000,000. However, no simple method is currently available for exploiting FACS approaches for isolating cells expressing soluble proteins based on the expression levels. A system that would allow the alternative production of membrane-anchored and soluble recombinant protein would therefore represent a valuable tool for the fast isolation of cells expressing very high protein levels.

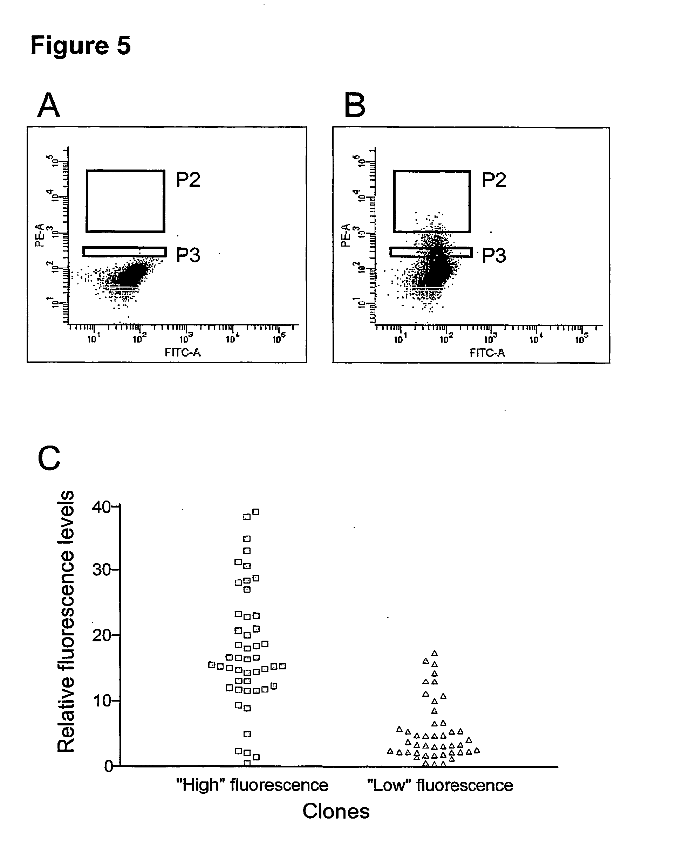

To demonstrate that a recombinant gene expression vector as disclosed herein can be used for the selection of cell clones producing very high recombinant protein levels, the retroviral vector *pLenti6-PC-UAAC-GPI* was used to transfect HEK293FT cells using the Lipofectamine™ 2000 (Invitrogen) transfection reagent. After 48 hours, supernatants containing retroviral particles were harvested, filter-sterilized to remove cell debris, and subsequently used to infect CHO-K1 cells. CHO-K1 cells were selected for resistance to the Blasticidin antibiotic at the concentration of 5 mg/l for 10 days. The resulting pools of Blasticidin-resistant cells were transferred into two culture flasks and grown to 25% confluency. To induce translational readthrough, the antibiotic G-418 was added to one culture flask at the final concentration of 100 mg/l and flasks were incubated for another 48 hours at 37°C. Cells were detached from the flasks by trypsinization and were incubated with mouse anti-human PC monoclonal antibodies. Cells were subsequently washed and incubated with a secondary antibody (rabbit anti-mouse IgG, Phycoerythrin (PE)-labeled (DAKO R0439)). Labeled CHO-K1 cells were sorted based on their relative fluorescence at 585 nm using a FACS Vantage™ cell sorter (Becton Dickinson) and using an excitation wave length of 488 nm. The results of FACS sorting of cells cultured in the presence of G-418 are shown in Figure 5B, while Figure 5A shows the results for cells cultured without G-418. Cells exhibiting high fluorescence levels (gate P2, Figure 5B) or moderate fluorescence levels (gate P3, Figure 5B) were individually sorted into 96-well cell culture plates containing 0.1 ml of culture medium without G-418, to allow the production of soluble recombinant PC. Cell culture plates were incubated at 37°C for 5 days, after which the presence of individual cell colonies in each culture well was assessed by microscopy. Plates were incubated at 37°C until cells reached confluency, after which cells were transferred to larger culture wells (12-well culture plates containing 1 ml of medium in each well). Cells were grown to 25% confluency, after which fresh medium containing G-418 at a final concentration of 100 mg/l was added to each well to induce translational readthrough. Culture plates were incubated for another 3 days at 37°C. Supernatants were saved and stored at -80°C until soluble PC activity assays were performed. Cells were trypsinized and then labeled with primary and secondary antibodies as described above, and subsequently analyzed for fluorescence using a FACScalibur™ cell analyzer (Becton Dickinson) with an excitation wave length of 488 nm and an emission filter of 585 nm.

Cells whose fluorescence was included in gate P2 or P3 of Figure 5B were individually sorted and grown further, after which analysis of membrane-anchored PC was performed by FACS. The results are presented in Figure 5C, which shows the detection of membrane-anchored PC in CHO-K1 clones expressing the PC-GPI fusion. 48 clones that exhibited low PC levels and 48 clones that exhibited high PC levels during FACS sorting were assayed for membrane-anchored PC levels by means of FACS analysis. The results confirm the relative recombinant protein expression levels that were observed during FACS sorting. Indeed, most clones that were sorted as high PC expressers exhibited higher recombinant protein levels than clones that were sorted as low PC expressers. These results indicate that the FACS sorting step was successful, both in measuring membrane-anchored PC levels and in the individual cell sorting.

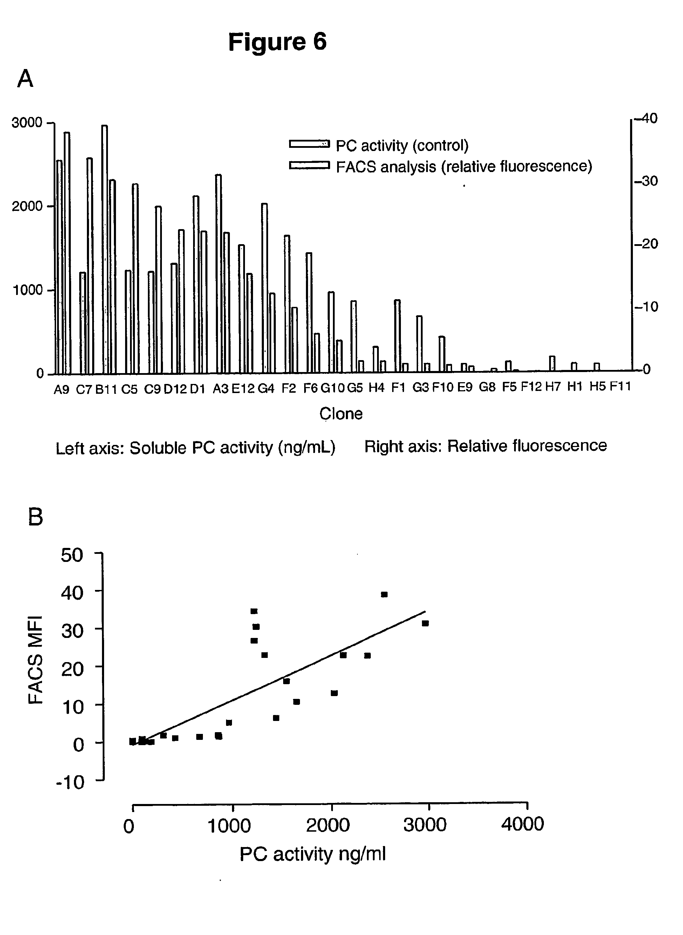

To assess whether there is a correlation between membrane-anchored (i.e. aminoglycoside-induced readthrough) and soluble (i.e. efficient translational termination after the PC sequence) PC levels, supernatants from 26 clones exhibiting various membrane-anchored PC levels were measured in an enzymatic-based PC assay. The results of this assay, presented in Figure 6A, confirm that there is a correlation between soluble and membrane-anchored recombinant protein expression levels. Indeed, clones exhibiting high membrane-anchored PC levels (as assessed by FACS analysis) exhibit high soluble PC levels whereas clones exhibiting low membrane-anchored PC levels exhibit relatively low soluble PC levels. FACS fluorescence levels and soluble PC activity levels were plotted on a new graph to further confirm the correlation between membrane-anchored and soluble recombinant protein expression levels (Figure 6B). Statistical analysis using a Pearson correlation test and assuming a Gaussian distribution indicates that there is less then 0.01 % chance (P value 0.0001) that the data are due to random distribution.

In conclusion, the data presented here show that there is a direct correlation between soluble and membrane-anchored PC levels. As a result, the present invention provides a high throughput (HTP) FACS-based method for the efficient selection of individual clones expressing high levels of soluble recombinant proteins.

### Example 4: Efficient selection of cell clones expressing high levels of recombinant Factor VII by FACS in serum-free conditions

Factor VII (FVII) is a zymogen for a vitamin K-dependent serine protease that is essential for the initiation of blood coagulation. FVII is a soluble protein that is primarily synthesized in the liver and that circulates in plasma. The FVII protein harbors distinct functional domains: the N-terminal domain, also known as Gla domain, is post-translationally modified by gamma-carboxylation of glutamic acid residues. Additionally, the FVII protein contains two domains with homology to epidermal growth factor (EGF1 and EGF2), and a C-terminal serine protease domain. Because of its important role in the treatment of hemostasis disorders, the recombinant FVII protein is produced in transgenic cells. However, in order to obtain an active molecule, the recombinant protein must be produced in transgenic cells exhibiting post-translational protein modification similar to the native molecule, namely mammalian cells. In contrast to bacterial or fungal heterologous production systems, yields of recombinant proteins synthesized in mammalian cell cultures are often low and associated with genomic instability of the transgene. Furthermore, most mammalian cell lines that are used for the heterologous expression of recombinant proteins have special nutritional requirements, such as the addition of mammalian serum (e.g. fetal bovine serum = FBS). Because such additives are of animal origin, this has raised concern about the possible presence of infectious agents such as retrovirus and prions.

Initial production of recombinant cell lines is traditionally performed in the presence of serum in the culture medium. After the isolation of clones producing desired levels of recombinant protein, the clones must be adapted to serum-free growth conditions prior to the production of therapeutic pharmaceutical proteins. This is a very labor intensive task that limits the number of clones that can be processed. Moreover, many clones do not achieve the desired recombinant protein expression levels after they have been adapted to serum-free conditions. We have developed a CHO-K1 cell line that does not require the addition of serum in the culture medium. The adaptation to serum-free conditions was performed by progressive reduction of the FBS concentration in the culture medium over a period of time (data not shown). This cell line (CHOK1-JRH325) is maintained in EX-CELL™ 325 PF CHO Serum-Free Medium (JRH325; JRH Biosciences), which is a chemically defined culture medium devoid of components of animal origin, and is therefore free of infectious agents. The serum-free -adapted CHOK1-JRH325 cell line is a non-adherent cell line which exhibits a similar growth rate to the parental CHO-K1 cell line.

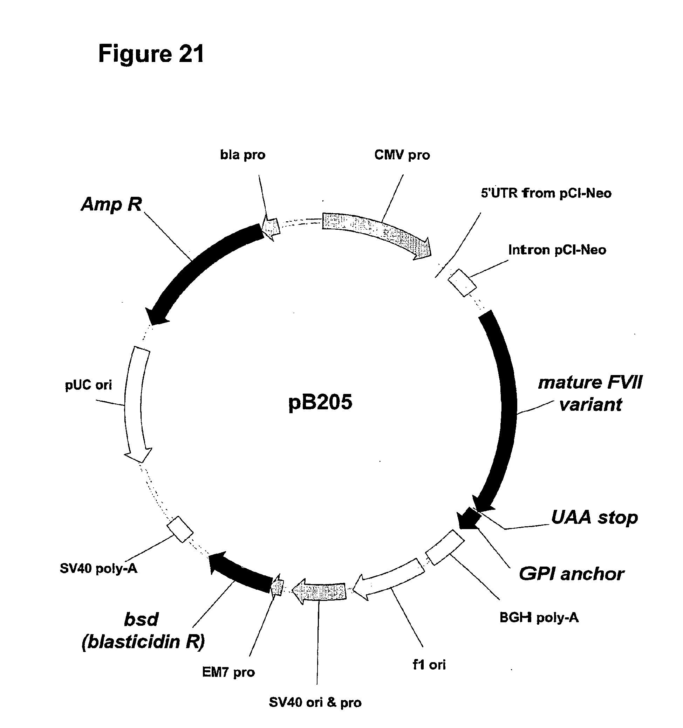

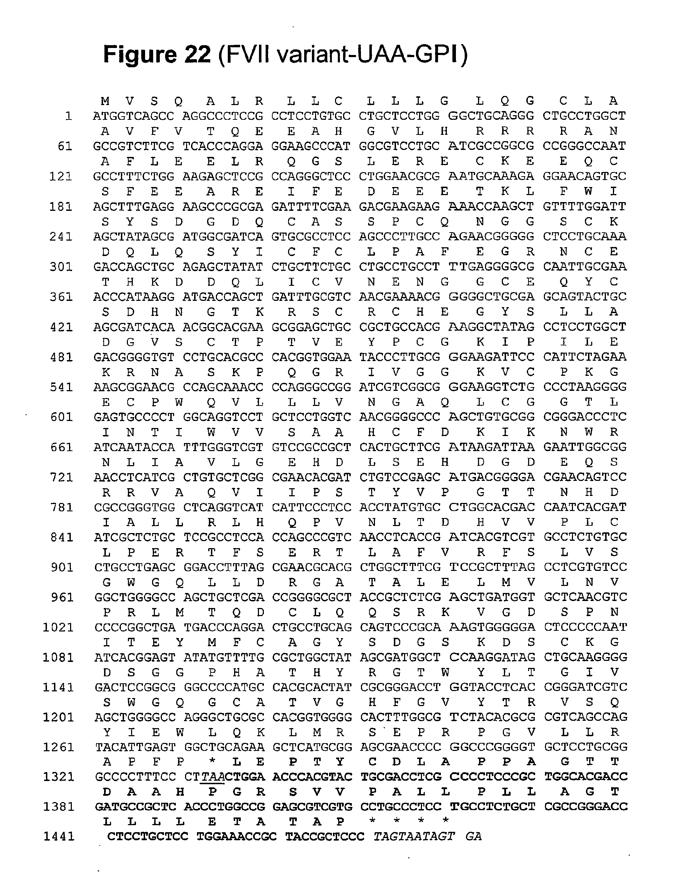

To demonstrate that a recombinant gene expression vector as disclosed herein can be used for the selection of mammalian cell clones producing very high recombinant FVII levels in serum-free growth conditions, the retroviral vector *pB205* was constructed (Figure 21). This vector harbors a translational fusion between a variant of human FVII (with the amino acid substitutions P10Q, K32E, A34E, R36E, T106N and V253N compared to wild-type human FVII), a UAA stop codon and the GPI anchoring signal (Figure 22). The translational fusion is under the transcriptional control of the CMV promoter. Additionally, the vector contains the *bsd* gene conferring resistance to the Blasticidin antibiotic. The *pB205* plasmid was used to transfect CHOK1-JRH325 cells using the FuGENE 6 (Roche Applied Science) transfection reagent. The cells were selected for resistance to the Blasticidin antibiotic at the concentration of 2.5 mg/l for 10 days. The resulting pool of Blasticidin-resistant cells was partly subjected to a "classical" dilution cloning procedure, partly to three rounds of translational readthrough with FACS sorting according to the invention.

The classical dilution procedure was aimed at seeding an individual cell in each well of 96-well culture plates. Cells were allowed to grow until the colonies covered most of the culture well area, after which approximately 370 clones were assayed in a first round of ELISA to select the clones expressing the highest levels of soluble FVII. From these 370 clones, the 44 clones with the highest expression levels were transferred to T-flasks for further growth and analysis (see below).

Alternatively, cells from the same original pool of Blasticidin-resistant cells transgenic for the B205 construct were treated with 100 mg/L Geneticin for 2 days to promote translational readthrough. The cells were harvested and incubated with a hybridoma-produced anti-human FVII mouse monoclonal antibody (mAB) targeted against the EGF1 domain of the FVII protein. The cells were subsequently washed and incubated with a secondary antibody (rabbit anti-mouse IgG, Phycoerythrin-labeled (DAKO R0439)). Labeled retroviral cell lines were analyzed by FACS for membrane-anchored recombinant FVII using a FACSVantage™ cell sorter (Becton Dickinson) with an excitation wave length of 488 nm and an emission filter of 585 nm. The cells exhibiting the highest fluorescent signal (best 10%; 1,000,000 cells sorted) were sorted and grown further.

The resulting sorted cell population was subjected to a second round of Geneticin treatment and FACS-based enrichment in which the cells exhibiting the highest fluorescent signal (best 3.5%; 400,000 cells sorted) were sorted as a pool and grown further. These cells were subjected to a third round of Geneticin treatment and FACS-based enrichment, with the cells exhibiting the highest fluorescent signal (best 4%; 50,000 cells sorted) being selected for further growth and analysis. These cells were allowed to grow for a few days, after which they were submitted to a "classical" dilution cloning procedure aiming at seeding an individual cell in each well of 96-well culture plates. Cells were allowed to grow until the colonies covered most of the culture well area, after which the cells were analyzed for recombinant soluble FVII protein levels by means of ELISA. Approximately 220 clones originating from the FACS-based enrichment and 370 clones originating from the "classical" limited dilution cloning as described above were assayed in a first round of ELISA to select the clones expressing the highest levels of soluble FVII. From these clones, 28 clones originating from the FACS-based enrichment and 44 clones originating from the "classical" limited dilution cloning were transferred to T-flasks and allowed to grow until the cells covered approximately half of the T-flask area, with regular medium replenishment. The cell density was measured for each clone simultaneously with a second ELISA-based measurement of the soluble recombinant FVII present in the culture medium. This allowed calculation of the specific productivity for each clone, determined as pg of FVII produced per cell daily (pg FVII/cell/day).

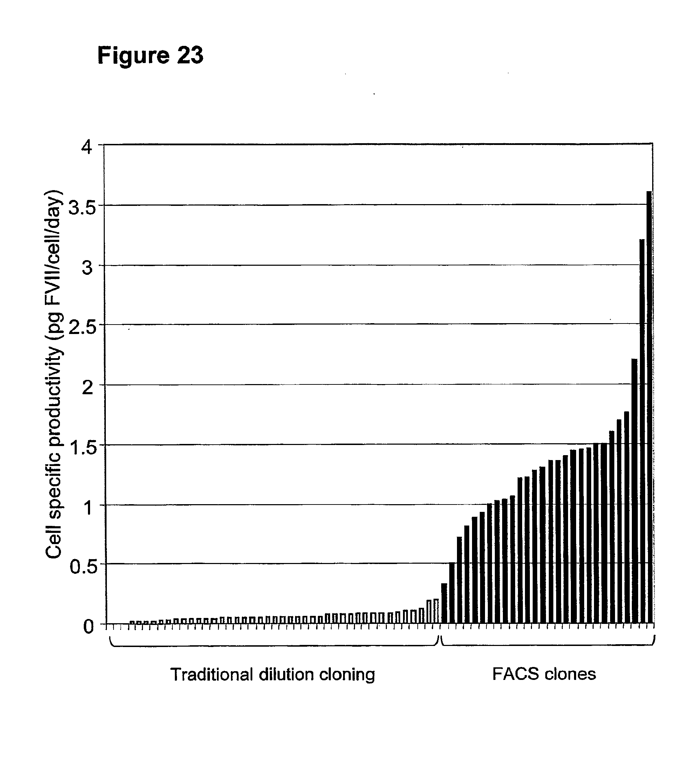

The results presented in Figure 23 show a comparison of production of soluble recombinant FVII between "classical" limited dilution cloning on the one hand and the Regulated Readthrough approach of the invention in conjunction with FACS capabilities on the other hand. The results unequivocally show that the clones that have been subjected to Regulated Readthrough and FACS ("FACS clones") secrete much larger amounts of recombinant FVII (average = 23 fold more) than the clones arising from the classical approach. Moreover, all of the FACS clones whose productivity has been assessed in Figure 23 exhibit higher recombinant protein productivity than the best clone obtained from classical limited dilution cloning. Taken together, these results confirm that the Regulated Readthrough technology, in conjunction with FACS capabilities, offers a powerful tool for the isolation of clones secreting very high levels of soluble recombinant protein in serum-free culture conditions.

### Example 5: Alternative production of soluble or membrane-anchored recombinant protein from the same cell - screening of functional libraries

Many downstream applications after fluorescence activated cell sorting (FACS) require the production of soluble recombinant protein. However, flow cytometry usually relies on the production of membrane- or intracellular-targeted recombinant protein. Therefore, the expression vectors to screen functional libraries typically include a membrane anchorage signal such as a GPI anchor or a transmembrane domain that will allow targeting of the recombinant protein to the cell membrane where it can be detected by flow cytometry. After the FACS-based enrichment of the library for clones exhibiting a desired trait (e.g. improved receptor-ligand binding), the recombinant DNA must be rescued and subcloned into a new vector for soluble protein expression (i.e. not containing a membrane anchoring signal). Thereafter, individual plasmid preparations are made prior to cell transfection and functional assays such as ELISA. This classical approach is time-consuming, requires robotic facilities and may result in the loss of some library diversity. Indeed, every manipulation of an expression library (PCR, ligation, cloning, transfection of target cells, etc.) results in the loss of library complexity. Additionally, the whole subcloning process is time-consuming and expensive. It would therefore be ideal to be able to produce a recombinant protein either as a soluble or membrane-anchored form from the same vector. In contrast to classical approaches, the "Regulated Readthrough" technology of the present invention makes it possible to perform the FACS-based screening and the functional analysis from the same cells.

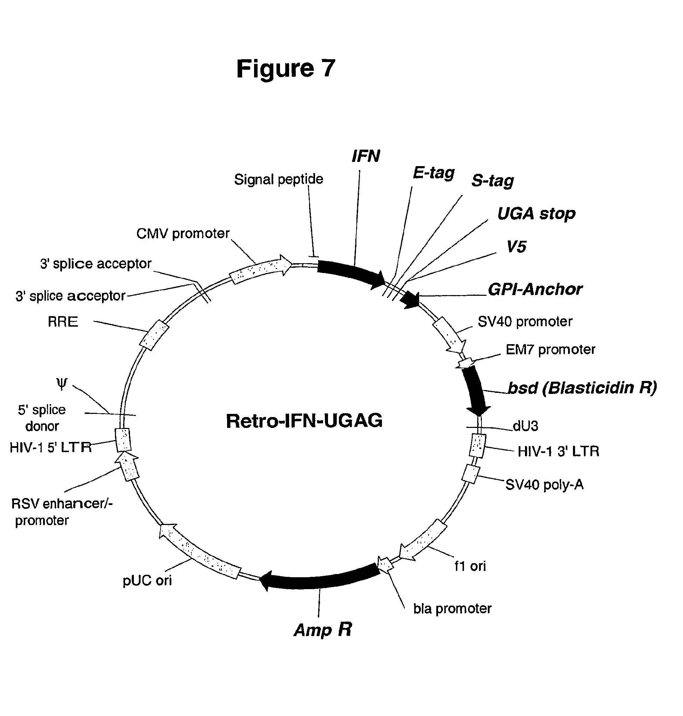

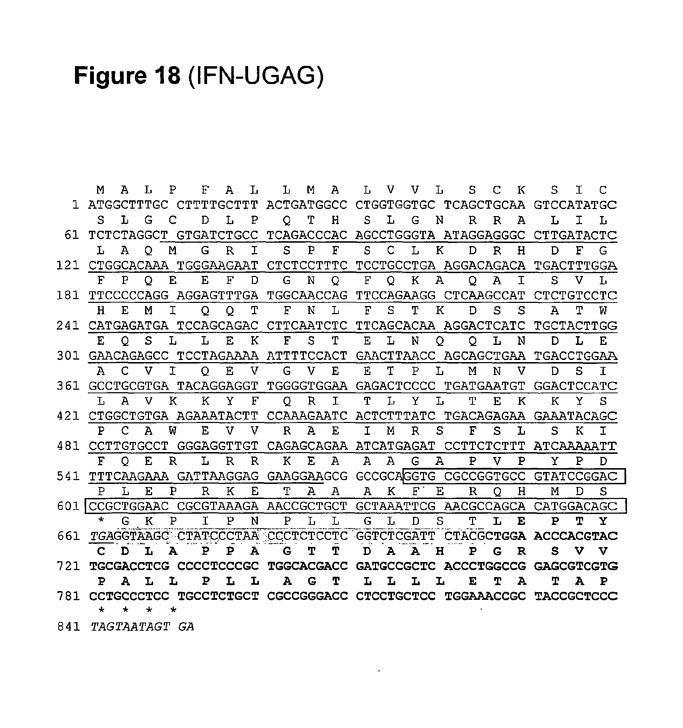

To demonstrate that a recombinant gene expression vector as disclosed herein can be used for the alternative production of soluble or membrane-anchored expression libraries, a human interferon alpha (IFNα) library may be created by molecular evolution (DNA shuffling), e.g. from 12 human genes coding for the IFNα family (Chang et al., 1999). The IFNα library is subcloned into a retroviral vector driving the expression of a cassette comprising a synthetic signal peptide, the IFNα sequence, the E-tag (amino acid sequence GAPVPYPDPLEPR) (SEQ ID NO:24) and S-tag (amino acid sequence KETAAAKFERQHMDS) (SEQ ID NO:25), the UGA nonsense codon (for example, the UGAC tetranucleotide), the V5 epitope and the GPI anchor. See Figure 7 for complete plasmid feature details, and Figure 18, which shows the coding sequence of a wild-type human IFN-alpha sequence, human IFN-alpha 21b, as well as other DNA and amino acid sequence details of the IFN-UGAG cassette. As shown in Figure 18, the IFN-UGAG cassette comprises a DNA sequence encoding a synthetic signal peptide, the IFN sequence, the E-tag, the S-tag, the UGA stop codon, the V5 epitope, and the GPI anchor (SP-IFN-Etag-Stag-UGA-V5-GPI).

The resulting library is used to transfect HEK293FT cells using the Lipofectamin™ 2000 (Invitrogen) transfection reagent. After 48 hours, supernatants containing retroviral particles are harvested, filter-sterilized to remove cell debris, and used to infect CHO-K1 cells. CHO-K1 cells stably transfected with the library are selected for resistance to the Blasticidin antibiotic at the concentration of 5 mg/l for 10 days. To induce translational readthrough, the antibiotic G-418 is added to the culture flask at a final concentration of 100 mg/l and the flask is incubated for another 48 hours at 37°C. The membrane-anchored protein fusion is detected using an FITC-labeled antibody targeted against the V5 epitope (Invitrogen 46-0308). The G-418 treatment is expected to substantially increase the percentage of cells displaying detectable levels of fusion protein. Further, the presence of a stop codon between the IFNα-E-tag-S-tag and the V5-GPI sequences is expected to result in a dramatic reduction in the percentage of FACS-positive cells in the unsorted original population as compared to a similar library that does not include this stop codon. Since stop codon readthrough is only partial, this reduction in FACS-positive cells among the original population will also be seen even in the presence of aminoglycoside. As a result, when using the Regulated Readthrough approach, it is preferable to use libraries exhibiting higher levels of diversity in order to provide a level of diversity similar to that of libraries that are displayed without this approach. This is acceptable for most expression libraries.

To further increase the percentage of cells displaying detectable levels of membrane-anchored fusion protein, the same experiment may be repeated by culturing the cells in the presence of about 2 mM sodium butyrate, which is often used to increase the expression levels of recombinant proteins (Gorman et al., 1983). In the presence of both G-418 and sodium butyrate, the percentage of cells displaying detectable levels of fusion protein is expected to increase substantially over the percentage obtained using G-418 alone.

The experiment may e.g. be performed by sorting as a pool one million cells that are positive for the V5 epitope display ("the V5 population"). It is expected that the cells will grow normally, and that the simultaneous treatment of the cells with G-418 and sodium butyrate and the FACS step will not affect the survival of the cells. The V5 population is treated with G-418, then analyzed for recombinant protein display using the FITC-labeled anti-V5 antibody. A high fraction of the population is expected to exhibit detectable levels of recombinant protein, as assessed by the fluorescence levels. To investigate the binding of the V5 population to a soluble, histidin-tagged truncated IFN receptor 2 (sIFNAR2-His), the cells may be incubated with e.g. 100 nM of receptor. The binding of sIFNAR2-His to the displayed IFN library may be detected by using a combination of mouse IgG1 anti-His and RPE-labeled, rat anti-mouse IgG1 antibodies. The FITC-labeled mouse IgG2 anti-V5 antibody is used to assess the levels of recombinant protein fusion displayed at the cell surface. It is expected that a relatively high percentage of the cells that are pretreated with G-418 will exhibit binding to sIFNAR2-His that is detectable by flow cytometry, and that the Regulated Readthrough technology will allow the FACS-based screening of expression libraries exhibiting a relatively high percentage of non-functional clones.

Following FACS sorting, independent cells (i.e. clones) are cultured in 96-well culture plates without G-418 to allow efficient translational termination and therefore promote the production of a soluble IFNα-E-tag-S-tag library. Cells are grown to confluency, after which supernatants are assayed for RNAase activity, which is mediated by the presence of the S-tag from the soluble IFNα-E-tag-S-tag chimeras.

### Example 6: Evaluation of heterogeneity of recombinant protein expression in cell populations by FACS analysis

The production of recombinant protein in mammalian cells for therapeutic use requires the isolation of clones producing stable recombinant protein levels throughout generations. Unfortunately, cells derived from the same original clones often exhibit substantial variations in recombinant protein expression levels. This can result from various causes, such as genetic instability or DNA methylation. As a result, recombinant cell lines that exhibit such discrepancies are inappropriate and must be discarded, in spite of their recombinant protein expression levels.

To demonstrate that a recombinant gene expression vector as disclosed herein can be used for the evaluation of heterogeneity of recombinant protein expression in cell clones, the retroviral vector *pLenti6-PC-UAAC-GPI* was used to transfect HEK293FT cells and produce retrovirus as described in Example 2. CHO-K1 cells were selected for resistance to the Blasticidin antibiotic at the concentration of 5 mg/l for 10 days. To induce translational readthrough, the antibiotic G-418 was added to the culture flask at the final concentration of 100 mg/l and the flask was incubated for another 48 hours at 37°C. Cells were detached from the flasks by trypsinization and were subsequently subjected to FACS sorting as described in Example 2. Individual cells were sorted based on their fluorescence levels in 96-well culture plates containing 0.1 ml of culture medium without G-418 to allow the production of soluble recombinant PC. Cell culture plates were incubated at 37°C for 5 days, after which the presence of individual cell colonies in each culture well was assessed by microscopy. Plates were incubated at 37°C until cells reached confluency and cells were subsequently transferred to larger culture wells (12-well culture plates containing 1 ml medium each). Cells were grown to 25% confluency, after which fresh medium containing G-418 at a final concentration of 100 mg/l was added to each well to induce translational readthrough. Culture plates were incubated for another 3 days at 37°C. Cells were trypsinized, then labeled with primary and secondary antibodies as described in Example 2, and subsequently analyzed for fluorescence using a FACScalibur™ cell analyzer (Becton Dickinson).

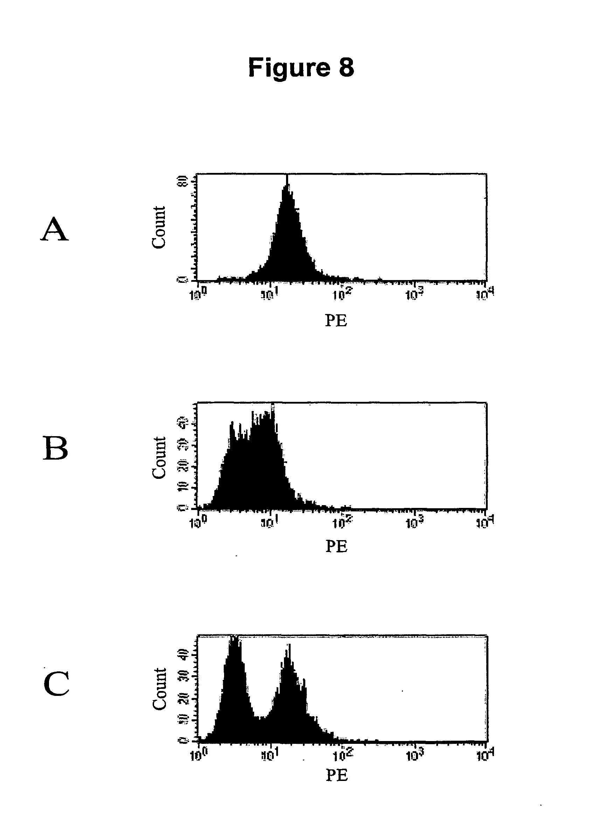

The results shown in Figure 8 confirm that the present invention allows the analysis of clones for uniformity of recombinant protein expression within the cell population. Indeed, FACS analysis of cell clones expressing the PC-GPI fusion reveals that some clones express relatively uniform PC-GPI protein levels (Figure 8A) whereas other clones exhibit much more variable recombinant protein levels that typically result in broader fluorescence peaks (Figure 8B). Although the latter may express similar overall recombinant protein levels, they are not suitable as producer cell lines because the overall recombinant expression levels usually drop throughout generations, because cells that have partially or totally lost the ability to produce recombinant protein generally grow faster than cells expressing high levels of recombinant protein.

Additionally, FACS-based cell cloning sometimes leads to mistakes that result in the presence of more than one cell in each cell culture well. The presence of multiple clones in the same well is generally assessed by microscopy but is labor-intensive and may lead to incorrect evaluations. An example of the presence of at least two different cell clones is presented in Figure 8C. Indeed, two clear individual peaks corresponding to cell populations expressing either low or high PC-GPI fusion protein levels are visible. The presence of two different cell populations may have arisen from failure at the FACS step, leading to the sorting of two cells in the same well. Alternatively, it is possible that the cell population expressing the lowest PC levels has arisen from cells that have lost the ability to express the recombinant protein. The potential causes of such loss of expression capability are multiple and may include chromosome rearrangements or DNA methylation. In any event, such cell populations have to be discarded.

Until now, these clones exhibiting discrepancies in recombinant protein expression levels were not distinguishable from clones exhibiting stable recombinant protein expression levels at early stages post-cloning. Usually, regular enzymatic measurement of recombinant protein levels for many cell culture generations is required to be able to identify and thus eliminate such unstable clones. This step is labor intensive and drastically reduces the number of clones that can be analyzed.

The present invention provides an inexpensive alternative method that can be performed at early stages to analyze the stability of recombinant protein expression levels. Additionally, the invention permits detection of the presence of multiple cell populations expressing different recombinant protein expression levels in putative cell clones.

### Example 7: Alternative production of tagged or native soluble recombinant protein from the same cell

Recombinant proteins that are expressed in eukaryotic cells are often translationally fused to epitope tags that are usually short peptides for which specific antibodies are available. Alternatively, larger peptides that exhibit interesting enzymatic or biochemical properties (reporter peptides) can be translationally fused to the protein of interest. Tagging of recombinant protein by translational fusion with epitope tags or larger peptides has multiple applications, including protein purification via affinity matrix (e.g. poly-Histidine tag, V5 epitope), subcellular localization (GFP variants), western blotting and immuno-precipitation (epitope tags).

However, the presence of peptide tags may interfere with the properties of the protein of interest, inhibiting protein folding, secretion, or enzymatic activities. Additionally, the presence of a tag may be toxic for the cell or be simply not desired in downstream applications. As a result, the presence of a peptide tag may be desired only transiently.

The present invention represents an ideal tool for the alternative production of recombinant proteins in their native or tagged forms from the same cells.

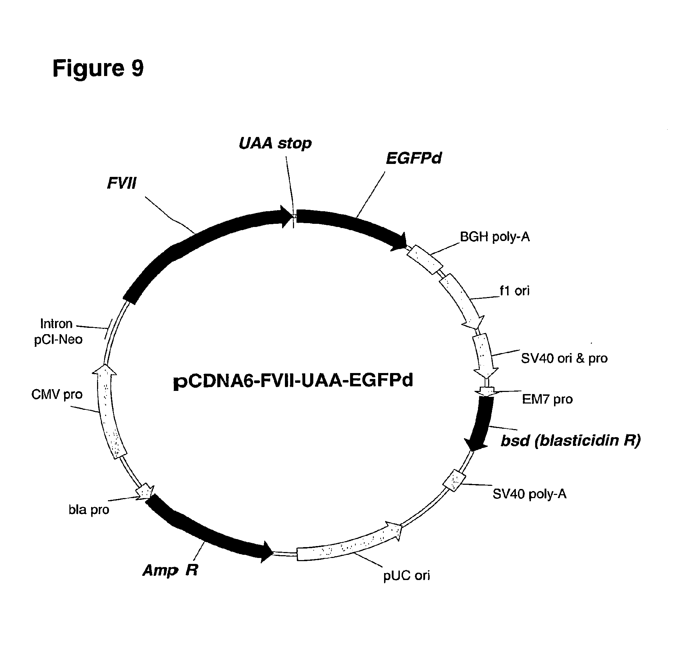

In the following example, the sequence encoding the human coagulation factor seven (FVII) is translationally fused to the sequence encoding the Enhanced Green Fluorescent Protein (EGFP) (GenBank Accession No. AAB02572) using a PCR approach similar to that described in Example 1. In order to avoid possible internal translation re-initiation, the first Methionine (Met) codon of the EGFP is removed and replaced by the UAA translation termination triplet. The resulting DNA fragment is cloned into the vector *pCDNA6*/*myc-His-A* (Invitrogen) to give the vector *pCDNA6-FVII-UAA-EGFPd,* which contains four termination stop codons downstream of the EGFP gene (Figure 9).

The vector *pCDNA6-FVII-UAA-EGFPd* is used to transfect CHO-K1 cells using the Lipofectamine™ 2000 (Invitrogen) transfection reagent. After 48 hours, cells are selected for resistance to the Blasticidin antibiotic at the concentration of 5 mg/l for 10 days. The resulting pools of Blasticidin-resistant cells are transferred into two culture flasks and grown to 25% confluency. To induce translational readthrough, the antibiotic G-418 is added to one culture flask at the final concentration of 100 mg/l and both flasks are incubated for another 48 hours at 37°C. Supernatants are harvested and assayed for the presence of FVII and EGFP proteins by ELISA and fluorescence assays, respectively.

In the presence of G-418, translational readthrough will occur and the EGFP reporter will be detected. In contrast, no EGFP fluorescence is expected above background levels in supernatants of cells grown in the absence of G-418. To confirm this result, a western blot using anti-FVII antibodies may be performed. A 45 kDa band is expected in supernatants from both G-418-treated and untreated samples. This band corresponds to the native FVII protein. A second band that exhibits a higher molecular size (72 kDa) is expected to be present only in supernatants from the G-418-treated cells. This larger band corresponds to a protein fusion comprising the FVII and the EGFP proteins.

### Example 8: Alternative production of tagged or native membrane-anchored recombinant protein from the same cell

Some recombinant proteins that are produced in cells are targeted to the plasma membrane. This is the case for many hormone receptors. Because these proteins are also anchored into the plasma membrane of the host cells, it is possible to enrich for cells expressing high recombinant protein levels using a FACS approach. However, this approach requires that specific antibodies to the receptor are available for the detection of the recombinant protein. Alternatively, chemicals or peptides that are known to interact specifically with the recombinant protein can be used. If none are available, the present invention represents an attractive alternative because epitope or peptide tags that are translationally fused to the recombinant protein can be expressed in aminoglycoside-treated cells.

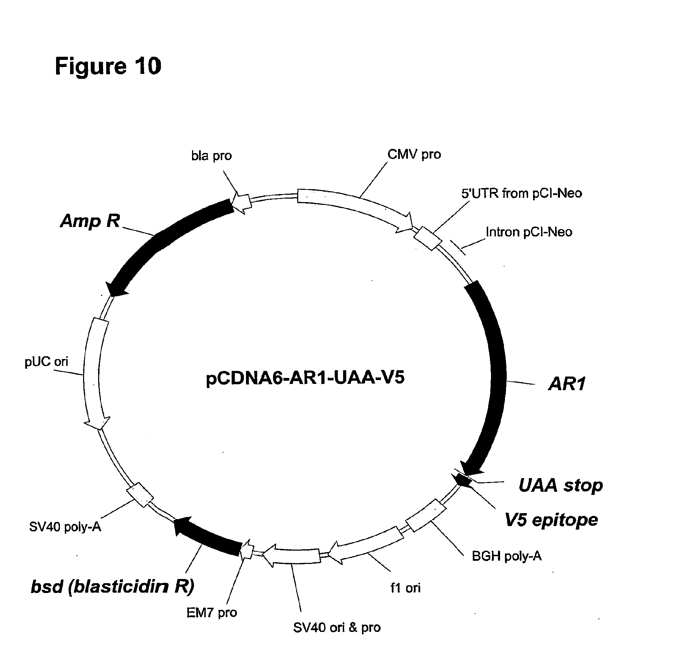

To demonstrate that the invention described herein can be used for the alternative production of tagged or native membrane-anchored recombinant protein from the same cell, the vector *pCDNA6-AR1-UAA-V5* is constructed (Figure 10). This vector drives the expression of the Adiponectin receptor 1 (AdipoR1) that belongs to the 7M transmembrane receptor family (Yamauchi et al., 2003). The *pCDNA6-AR1-UAA-V5* vector contains a UAA stop codon immediately downstream of the AdipoR1 sequence, as well as a sequence coding for the V5 epitope.

CHO-K1 cell lines stably transfected with this vector are generated as described in Example 5. Following generation of cell lines, cells are divided into two flasks and grown to 25% confluency. To induce translational readthrough, the antibiotic G-418 is added to one culture flask at the final concentration of 100 mg/l, then both flasks are incubated for 48 hours at 37°C. Cells are detached from the flasks by trypsinization and are subsequently incubated with FITC-labeled anti-V5 monoclonal antibodies (Invitrogen 46-0308).

Labeled CHO-K1 cells are sorted based on their relative fluorescence at 530 nm using a FACSVantage™ cell sorter (Becton Dickinson) with an excitation wave length of 488 nm. Cells exhibiting high or moderate fluorescence levels are individually sorted into 96-well cell culture plates containing 0.1 ml of culture medium without G-418, to allow the production of recombinant native AdipoR1. Cell culture plates are incubated at 37°C for 5 days, after which the presence of individual cell colonies in each culture well is assessed by microscopy. Plates are incubated at 37°C until cells reach confluency and cells are subsequently transferred to larger culture wells (12-well culture plates containing 1 ml medium each). Cells are grown to 25% confluency, after which fresh medium containing G-418 at a final concentration of 100 mg/l is added to each well to promote translational readthrough. Culture plates are incubated for another 3 days at 37°C. Cells are trypsinized, then labeled with anti-V5 antibody as described above, and subsequently analyzed for fluorescence using a FACScalibur™ cell analyzer (Becton Dickinson).

48 clones that exhibit low V5 levels and 48 clones that exhibit high V5 levels during FACS sorting are assayed for membrane-anchored V5 levels by means of FACS analysis. These results are expected to confirm the relative recombinant protein expression levels that are observed during FACS sorting. It is expected that most clones that are sorted as high V5 expressers will exhibit higher recombinant protein levels than clones that are sorted as low V5 expressers.

As a result, the present invention provides a high throughput (HTP) FACS-based method for the efficient selection of individual clones expressing high levels of membrane-anchored recombinant proteins.

### Example 9: Selection of recombinant cell lines devoid of antibiotic resistance

To obtain cell lines producing a recombinant protein of interest, classical methods rely on the presence of an additional recombinant gene that is carried by the DNA vector used during the transfection and that confer resistance to an antibiotic. After transfection, cells are cultivated in the presence of antibiotic concentrations known to inhibit cell growth or kill wild-type cells. As a result, only cells that express the recombinant protein conferring resistance to the given antibiotic are able to grow.

Although the presence of the resistance marker provides a valuable method for selecting cells expressing a recombinant protein of interest, many downstream applications do not require the presence, or the expression, of this selectable marker. For example, the promoter driving the resistance marker gene is often a very strong promoter of viral origin that is constitutively active. As a result, the recombinant RNA coding for the selection marker may compete with other RNAs for protein production and may reduce the yields of the recombinant protein of interest. Furthermore, the massive production of RNA coding for the selection marker may trigger post-transcriptional gene silencing, and therefore may lead to reduced yields of the recombinant protein of interest. Another advantage of a method enabling the selection of cell lines devoid of antibiotic resistance is that it would eliminate the potential for horizontal transfer of the antibiotic resistance selection marker gene to wild-type species, which represents a possible biohazard risk for the environment. A further potential advantage of the present invention is the possibility to create transgenic lines simultaneously expressing an unlimited number of different transgenes. Indeed, only a few selection markers are available to date, which limits drastically the number of different transgenes that can be expressed in the same cell.

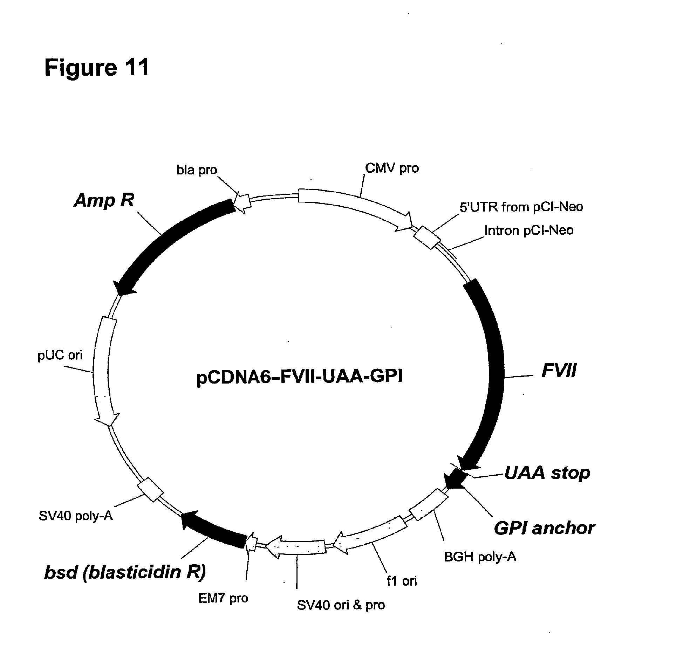

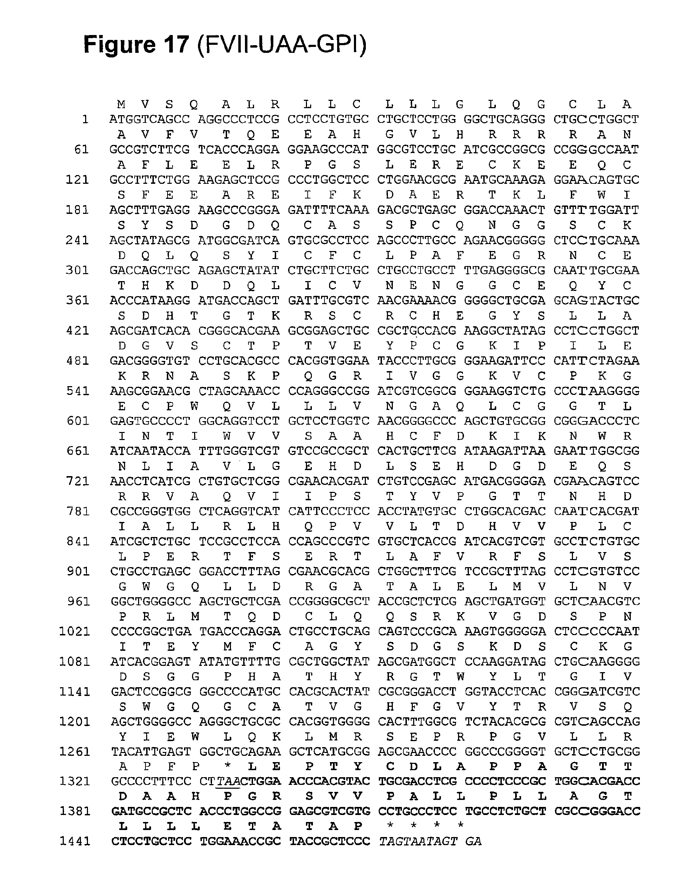

To demonstrate that the invention described herein can be used for the selection of recombinant cell lines devoid of antibiotic resistance, PCR was performed using the oligonucleotides TBO235 (5' AAGAATCTGCTTAGGGTTAGGCG 3') (SEQ ID NO:26) and TBO260 (5' CCTGCTATTGTCTTCCCAATCC 3') (SEQ ID NO:27) using the vector *pCDNA 6-FVII-UAA-GPI* (see Figure 17) as a template. The resulting PCR product encompassed the CMV promoter, the b-globin intron, the FVII gene, the UAA stop codon, the GPI anchor signal and the b-globin poly-adenylation signal (Figure 11). The PCR product was purified and subsequently used to transfect CHO-K1 cells with Lipofectamine™ 2000 (Invitrogen) as described above. Additionally, a flask containing CHO-K1 cells was incubated with Lipofectamine™ 2000 but without DNA as a negative control. Five hours after transfection, G-418 was added to the two culture flasks at a final concentration of 100 mg/L to promote translational readthrough. The cells were detached from the flasks using Cell Dissociation Solution (Sigma) and were incubated with mouse anti-human FVII monoclonal antibodies. The cells were subsequently washed and incubated with a secondary antibody (rabbit anti-mouse IgG, Phycoerythrin-labeled (DAKO R0439)). Labeled retroviral cell lines were analyzed by FACS for membrane-anchored recombinant FVII using a FACSVantage™ (Becton Dickinson) instrument with an excitation wave length of 488 nm and an emission filter of 585 nm.

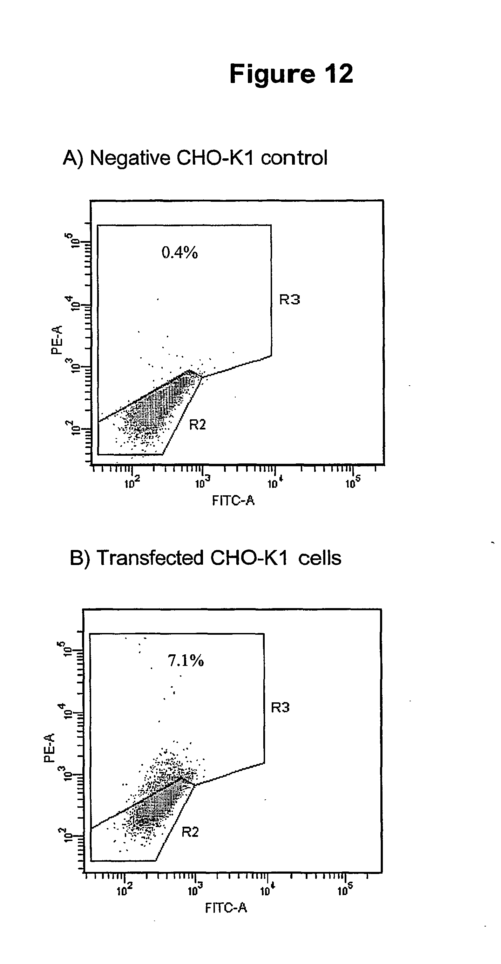

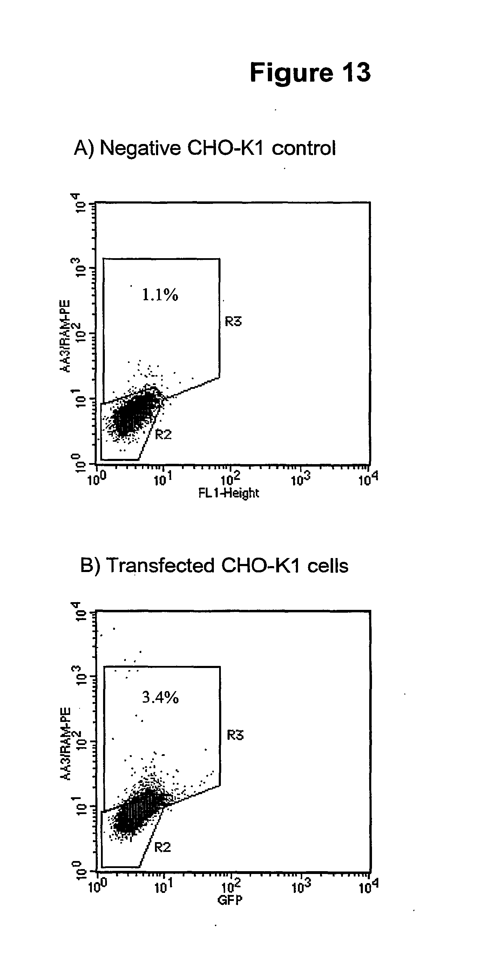

Because expression of the recombinant FVII protein is correlated with expression of the FVII-GPI protein fusion arising from aminoglycoside-mediated translational readthrough, transgenic cells can be selected by means of FACS based on membrane-anchored FVII detection. As shown in Figure 12, a clear population (gate R3; 7.1%) of transfected CHO-K1 cells exhibited fluorescence signals, as compared to only 0.4% for the negative control sample (where the 0.4% for the negative control cells is due to false positive background). Approximately 2500 cells from gate R3 of the transfected cells were sorted as a pool and grown for 9 days in the absence of antibiotic, after which translational readthrough was induced by treating the cells with G-418 at 100 mg/L for 2 days. The cells were detached from the flasks, then labeled for FVII detection and analyzed by flow cytometry as described above. Non-transfected CHO-K1 cells were grown in a similar manner as a negative control. As shown in Figure 13, the G-418 treatment resulted in 3.4% cells that were positive for FVII display, whereas only 1.1% of the cells were positive for the non-transfected CHO-K1 control, the latter again being due to false positive background.

It is interesting to note that a greater percentage of the transfected cells were positive for FVII display during the first round of FACS than during the second sorting step (7.1 % in the first round as compared to 3.4% in the second round). This result suggests that a substantial proportion of the cells that were positive for FVII display during the first sorting round did not stably integrate the transgene into their genome. This was expected because the first sorting round took place only 2 days post transfection. As a result, much of the recombinant protein that was detected was arising from transient expression due to the presence of the recombinant DNA used for the transfection. In contrast, when the second sorting round was performed, the cells had been cultured for a total of 13 days since they had been transfected. Hence they had either lost or stably integrated the transgene into their genome. Taken together, these results demonstrate that the present invention can be used to generate and select stable cell lines expressing recombinant proteins without the requirement for using a selection marker such as an antibiotic or a fluorescent protein.

Once a pool of cells stably expressing the recombinant protein has been obtained, it is possible to subject the cell pool to individual cell cloning by means of FACS or other methods.

### Example 10: Expression and screening of antibody libraries using the Regulated Readthrough approach

Monoclonal antibodies (mAB) are rapidly becoming one of the most common class of therapeutic proteins because of their high specificity to many classes of target antigens (Ag). Because full-length mABs are normally secreted into the culture medium of production cell lines, single-chain variable region fragment (scFv) have been developed to display the antibody fragment at the surface of bacteriophage particles. The phage display approach has been extensively used to enrich scFv antibody libraries for the binding to a given Ag. However scFv fragments obtained from such a screening procedure have to be grafted back into antibody light chain and heavy chain backbones prior to stable production in mammalian cell lines. This subcloning step is technically difficult, time-consuming and may result insome loss of specificity of the antibody because the scFv antibodies do not always preserve the binding specificity of complete antibodies. Furthermore, some mABs generated from such methods have proven difficult to produce at satisfactory concentration levels.

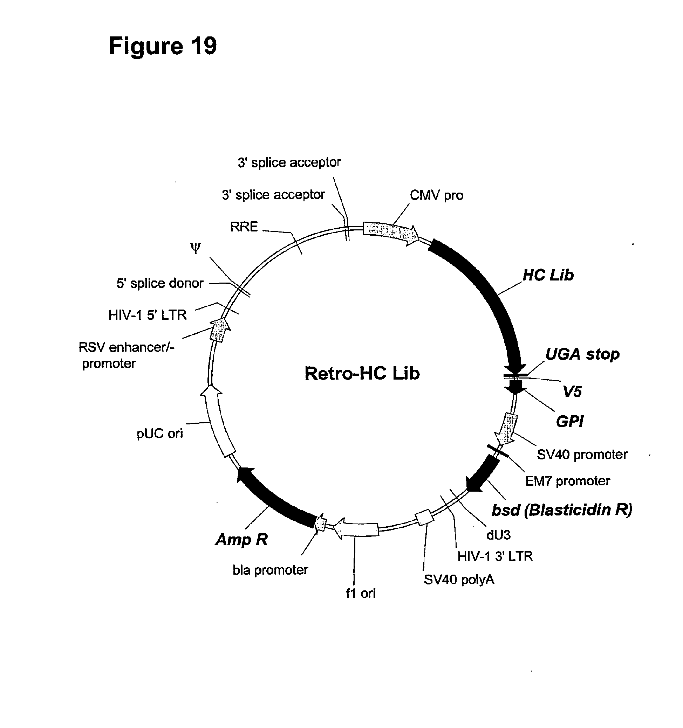

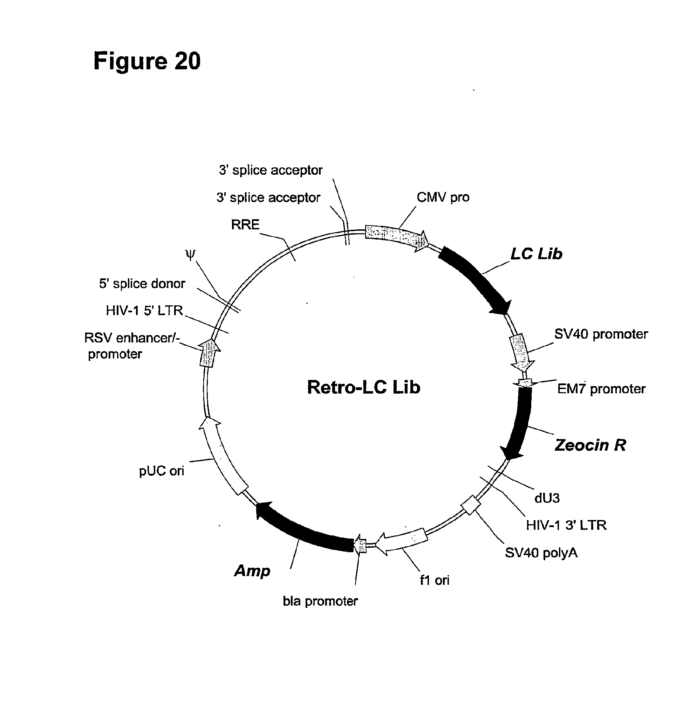

The Regulated Readthrough approach offers the unique opportunity to display full-length mABs at the cell surface of mammalian cell lines for FACS-based enrichment. In the following example, a full-length human antibody library is constructed by DNA shuffling, site-directed mutagenesis, or error-prone PCR. Two independent retroviral vectors exhibiting different antibiotic resistance markers are constructed to produce the mAB light chain library (LC lib) and heavy chain library (HC lib), as shown in Figures 19 and 20. Additionally, the vector for HC production contains a stop codon, a V5 epitope and a GPI anchoring signal. After generation of stable Blasticidin-resistant CHO-K1 cells lines expressing the Retro-HC Lib-STOP-V5-GPI cassette, the cells are enriched for HC and/or V5 display by means of flow cytometry after induction of translational readthrough by an aminoglycoside treatment. The sorted cells are subsequently infected with the second retroviral vectors for LC expression (Retro-LC Lib) and a stable pool is generated using Zeocin selection. A retroviral vector, pLenti4/V5-DEST, carrying the zeocin resistance gene is available from Invitrogen. The resulting cell population is treated with an aminoglycoside to promote translational readthrough. Thereafter the cell population is enriched for cells displaying detectable levels of full length mAB using a combination of fluorescent antibodies suitable for flow cytometry and targeted against the V5 epitope and the constant HC and LC domains of the displayed mAB.

Following aminoglycoside treatment, the library is simultaneously analyzed for Ag binding and mAB display (using a labeled antibody targeted against the V5 epitope, or HC) and subjected to a first round of enrichment aimed at sorting all cells that display mAB and that interact with the Ag. Several approaches for the detection of the mAB-Ag interaction are possible, depending on the nature of the Ag labeling. For biotin-conjugated Ag, a streptavidin-RPE detection step allows visualization of the fluorescence at 585 nm. For fluorescein-labeled Ag, the fluorescence is visualized at 530 nm. An anti-V5 antibody is simultaneously used with the Ag detection as a marker for the amount of recombinant mAB displayed at the cell surface. The sorted cells are subsequently subjected to 1 or 2 rounds of off-rate-based enrichment using unlabeled Ag as a competitor and in the presence of an aminoglycoside to promote translational readthrough. The cells exhibiting a non-displaceable binding to the Ag are submitted to a last round of flow cytometry and individually cloned in 96-well culture plates. Because a stop codon is present immediately downstream of the HC, most of the HC produced in the absence of aminoglycoside will not have the V5-GPI tag and will therefore follow the secretory pathway. As a result, functional mAB will be secreted into the culture medium, thus allowing functional characterization directly from the supernatants of the sorted clones.

While the foregoing invention has been described in some detail for purposes of clarity and understanding, it will be clear to one skilled in the art from a reading of this disclosure that various changes in form and detail can be made.

For example, all the techniques, methods, compositions, apparatus and systems described above may be used in various combinations.

### REFERENCES

Bedwell et al., Nature Med. 3:1280-1284, 1997.
Barton-Davis et al., J Clin Invest. 104:375-381, 1999.
Burke & Mogg, Nucleic Acids Res. 13(17): 6265-72, 1985.
Chang et al., Nat Biotechnol. 17(8):793-797, 1999
Gorman et al., Nucleic Acids Res. 11(21):7631-48, 1983
Howard et al., Nature Med. 2:467-469, 1996.
Hunt et al., Biotechnol Bioeng. 77(5):528-37, 2002.
Ikezawa, Bull. Pharm. Bull. 25(4):409-417,2002
Kawagishi et al., Genomics 30(2):224-32, 1995
Kozak, J Mol Biol 196(4):947-50, 1987
Kurtzman et al., Curr Opin Biotechnol. 12(4):361-70, 2001
Manuvakhova et al., RNA 6(7): 1044-55, 2000
Millan, J. Biol. Chem. 261(7):3112-3115,1986
Pack et al., J Mol Biol 246:28-34, 1995
Pack et al., Biotechnol 11:1271-77, 1993
Pack & Plueckthun., Biochemistry 31:1579-84, 1992
Palmer et al., Nature 277(5692): 148-50,1979.
Stansfield et al., EMBO J. 14:4365-4373, 1995.
Whalen et al., Curr Opin Mol Ther. 3(1):31-6,2001
Yamauchi et al., Nature 423(6941): 762-9, 2003
Zhouravleva et al., EMBO J. 14(16): 4065-72, 1995

## Claims

1. A method for screening or selecting cells expressing a desired level of a polypeptide, comprising:
a) providing a plurality of eukaryotic cells each comprising an expression cassette comprising a first polynucleotide encoding the polypeptide; at least one stop codon downstream of the first polynucleotide, and a second polynucleotide encoding a cell membrane anchoring peptide downstream of the stop codon;
b) cultivating the cells in the presence of a termination suppression agent under conditions that allow expression of the polypeptide, wherein the termination suppression agent is an aminoglycoside antibiotic; and
c) using flow cytometry to select at least one cell expressing the polypeptide fused to a cell membrane anchoring peptide.

2. A method for evaluating recombinant polypeptide expression in a population of cells, comprising:
a) providing a plurality of eukaryotic cells each comprising an expression cassette comprising a first polynucleotide encoding a recombinant polypeptide, at least one stop codon downstream of the first polynucleotide, and a second polynucleotide encoding a cell membrane anchoring peptide downstream of the stop codon;
b) cultivating the cells in the presence of a termination suppression agent under conditions that allow expression of a fusion protein comprising the recombinant polypeptide and the cell membrane anchoring peptide, wherein the termination suppression agent is an aminoglycoside antibiotic; and
c) sorting the cells by flow cytometry to select at least one cell expressing the fusion protein at a desired level and/or with a desired uniformity.

3. A method for screening or selecting at least one cell expressing a polypeptide with a desired binding affinity to a ligand from cells expressing a library of polypeptide variants, comprising:
a) providing a plurality of eukaryotic cells each comprising an expression cassette comprising a first polynucleotide encoding a polypeptide variant, at least one stop codon downstream of the first polynucleotide, and a second polynucleotide encoding a cell membrane anchoring peptide downstream of the stop codon;
b) cultivating the cells in the presence of a termination suppression agent under conditions that allow expression of the polypeptide variant, wherein the termination suppression agent is an aminoglycoside antibiotic; and
c) using flow cytometry to select at least one cell expressing the polypeptide variant fused to a cell membrane anchoring peptide based on binding affinity of said polypeptide variant to said ligand.

4. The method of any of the preceding claims, further comprising:
d) cultivating at least one selected cell in the absence of a termination suppression agent to obtain expression of the polypeptide as a soluble polypeptide.

5. A method for alternately expressing i) a soluble polypeptide or ii) a membrane-bound polypeptide from a single cell or cell line, comprising:
a) providing a plurality of eukaryotic cells each comprising an expression cassette comprising a first polynucleotide encoding the polypeptide, at least one stop codon downstream of the first polynucleotide, and a second polynucleotide encoding a cell membrane anchoring peptide downstream of the stop codon;
b) cultivating the cells in the presence of a termination suppression agent under conditions that allow expression of the polypeptide, wherein the termination suppression agent is an aminoglycoside antibiotic;
c) using flow cytometry to select at least one cell expressing the polypeptide fused to a cell membrane anchoring peptide; and
d) cultivating said selected cell in the absence of a termination suppression agent to obtain expression of the polypeptide as a soluble polypeptide.

6. The method of any of the preceding claims, wherein the at least one selected cell expresses a fusion protein comprising the polypeptide fused to a cell membrane anchoring peptide, the fusion protein being displayed at the surface of said cell.

7. The method of claim 6, wherein the cell membrane anchoring peptide is a GPI anchor.

8. The method of any of the preceding claims, wherein the aminoglycoside antibiotic is selected from the group consisting of G-418, gentamicin (gentamycin), paromomycin, hygromycin, amikacin, kanamycin, neomycin, netilmicin, paromomycin, streptomycin and tobramycin.

9. A method for alternately expressing i) a membrane-bound, untagged polypeptide or ii) a membrane-bound, tagged polypeptide from a single cell or cell line, comprising:
a) providing a plurality of eukaryotic cells each comprising an expression cassette comprising a first polynucleotide encoding the polypeptide and a cell membrane anchoring peptide, at least one stop codon downstream of the first polynucleotide, and a second polynucleotide encoding a reporter peptide or an epitope tag downstream of the stop codon;
b) cultivating the cells in the presence of a termination suppression agent under conditions that allow expression of the polypeptide and the cell membrane anchoring peptide, wherein the termination suppression agent is an aminoglycoside antibiotic;
c) using flow cytometry to select at least one cell expressing a fusion protein comprising the polypeptide, the cell membrane anchoring peptide, and a reporter peptide or an epitope tag; and
d) cultivating said selected cell in the absence of a termination suppression agent to obtain expression of a protein comprising the polypeptide in membrane-bound form without the reporter peptide or epitope tag.

10. The method of claim 9, wherein the cell membrane anchoring peptide is a GPI anchor.

11. The method of claim 9 or 10, wherein the second polynucleotide encodes a reporter peptide selected from the group consisting of green fluorescent protein, luciferase, β-galactosidase, β-glucuronidase and chloramphenicol acetyltransferase (CAT).

12. The method of claim 9 or 10, wherein the second polynucleotide encodes an epitope tag selected from the group consisting of V5, His, FLAG™, HA, c-Myc, VSV-G, and HSV.

13. A method for producing a polypeptide, comprising cultivating a cell line obtained by the method of any of the preceding claims, wherein the cell line is cultivated in the absence of an aminoglycoside antibiotic to allow expression of the polypeptide, and isolating said polypeptide.

14. The method of claim 13, where the polypeptide is a soluble polypeptide that is secreted into a culture medium, and the polypeptide is isolated from said medium.

15. A kit suitable for performing the method of any of the preceding claims, comprising one or more of: (1) at least one kit component comprising an expression cassette as defined in any of claims 1-12; a cell or expression vector comprising said expression cassette; an aminoglycoside antibiotic; or a composition comprising at least one such component; (2) instructions for practicing a method as defined in any of claims 1-14, instructions for using any component identified in (1) or any composition of any such component; (3) a container for holding said at least one such component or composition, and (4) packaging materials.

## Patentansprüche

1. Verfahren zum Screenen oder Auswählen von Zellen, die einen gewünschten Level eines Polypeptids exprimieren, umfassend:
a) Bereitstellen einer Vielzahl von eukaryotischen Zellen, die jede eine Expressionskassette, umfassend ein erstes Polynukleotid, welches das Polypeptid kodiert, mindestens ein Stopcodon stromabwärts des ersten Polynukleotids und ein zweites Polynukleotid, das ein Zellmembran-Ankerpeptid stromabwärts des Stopcodons kodiert, umfassen,
b) Kultivieren der Zellen in Anwesenheit eines die Termination unterdrükkenden Mittels bei Bedingungen, welche die Expression des Polypeptids erlauben, wobei das die Termination unterdrückende Mittel ein Aminoglycosid-Antibiotikum ist, und
c) Verwenden von Durchflußzytometrie, um mindestens eine Zelle auszuwählen, die das Polypeptid fusioniert mit einem Zellmembran-Ankerpeptid exprimiert.

2. Verfahren zum Bewerten rekombinanter Polypeptidexpression in einer Zellpopulation, umfassend:
a) Bereitstellen einer Vielzahl von eukaryotischen Zellen, die jede eine Expressionskassette, umfassend ein erstes Polynukleotid, welches ein rekombinantes Polypeptid kodiert, mindestens ein Stopcodon stromabwärts des ersten Polynukleotids und ein zweites Polynukleotid, das ein Zell-membran-Ankerpeptid stromabwärts des Stopcodons kodiert, umfassen,
b) Kultivieren der Zellen in Anwesenheit eines die Termination unterdrükkenden Mittels bei Bedingungen, welche die Expression eines Fusionsproteins, umfassend das rekombinante Polypeptid und das Zellmembran-Ankerpeptid, erlauben, wobei das die Termination unterdrückende Mittel ein Aminoglycosid-Antibiotikum ist, und
c) Sortieren der Zellen durch Durchflußzytometrie, um mindestens eine Zelle auszuwählen, welche das Fusionsprotein bei einem gewünschten Level und/oder mit einer gewünschten Gleichförmigkeit exprimiert.

3. Verfahren zum Screenen oder Auswählten mindestens einer Zelle, die ein Polypeptid mit einer gewünschten Bindungsaffinität zu einem Liganden exprimiert, aus Zellen, die eine Bibliothek von Polypeptidvarianten exprimieren, umfassend:
a) Bereitstellen einer Vielzahl von eukaryotischen Zellen, die jede eine Expressionskassette, umfassend ein erstes Polynukleotid, welches eine Polypeptidvariante kodiert, mindestens ein Stopcodon stromabwärts des ersten Polynukleotids und ein zweites Polynukleotid, das ein Zellmembran-Ankerpeptid stromabwärts des Stopcodons kodiert, umfassen,
b) Kultivieren der Zellen in Anwesenheit eines die Termination unterdrükkenden Mittels bei Bedingungen, welche die Expression der Polypeptidvariante erlauben, wobei das die Termination unterdrückende Mittel ein Aminoglycosid-Antibiotikum ist, und
c) Verwenden von Durchflußzytometrie, um mindestens eine Zelle auszuwählen, welche die Polypeptidvariante fusioniert mit einem Zellmembran-Ankerpeptid exprimiert, basierend auf der Bindungsaffinität der Polypeptidvariante zu dem Liganden.

4. Verfahren nach einem der vorhergehenden Ansprüche, weiter umfassend:
d) Kultivieren mindestens einer ausgewählten Zelle in Abwesenheit eines die Termination unterdrückenden Mittels, um Expression des Polypeptids als lösliches Polypeptid zu erhalten.

5. Verfahren zum abwechselnden Exprimieren i) eines löslichen Polypeptids oder ii) eines membrangebundenen Polypeptids von einer einzelnen Zelle oder Zelllinie, umfassend:
a) Bereitstellen einer Vielzahl von eukaryotischen Zellen, die jede eine Expressionskassette, umfassend ein erstes Polynukleotid, welches das Polypeptid kodiert, mindestens ein Stopcodon stromabwärts des ersten Polynukleotids und ein zweites Polynukleotid, das ein Zellmembran-Ankerpeptid stromabwärts des Stopcodons kodiert, umfassen,
b) Kultivieren der Zellen in Anwesenheit eines die Termination unterdrükkenden Mittels bei Bedingungen, die Expression des Polypeptids erlauben, wobei das die Termination unterdrückende Mittel ein Aminoglycosid-Antibiotikum ist,
c) Verwenden von Durchflußzytometrie, um mindestens eine Zelle auszuwählen, die das Polypeptid fusioniert mit einem Zellmembran-Ankerpeptid exprimiert, und
d) Kultivieren der ausgewählten Zelle in Abwesenheit eines die Termination unterdrückenden Mittels, um Expression des Polypeptids als lösliches Polypeptid zu erhalten.

6. Verfahren nach einem der vorhergehenden Ansprüche, wobei die mindestens eine ausgewählte Zelle ein Fusionsprotein exprimiert, umfassend das Polypeptid fusioniert an ein Zellmembran-Ankerpeptid, wobei das Fusionsprotein an der Oberfläche der Zelle präsentiert wird.

7. Verfahren nach Anspruch 6, wobei das Zellmembran-Ankerpeptid ein GPI-Anker ist.

8. Verfahren nach einem der vorhergehenden Ansprüche, wobei das Aminoglycosid-Antibiotikum ausgewählt ist aus der Gruppe, bestehend aus G-418, Gentamicin (Gentamycin), Paromomycin, Hygromycin, Amikacin, Kanamycin, Neomycin, Netilmicin, Paromomycin, Streptomycin und Tobramycin.

9. Verfahren zum abwechselnden Exprimieren i) eines membrangebundenen, unmarkierten Polypeptids oder ii) eines membrangebundenen, markierten Polypeptids von einer einzelnen Zelle oder Zelllinie, umfassend:
a) Bereitstellen einer Vielzahl von eukaryotischen Zellen, die jede eine Expressionskassette, umfassend ein erstes Polynukleotid, welches das Polypeptid und ein Zellmembran-Ankerpeptid kodiert, mindestens ein Stopcodon stromabwärts des ersten Polynukleotids und ein zweites Polynukleotid, das ein Reporterpeptid oder eine Epitop-Markierung stromabwärts des Stopcodons kodiert, umfassen,
b) Kultivieren der Zellen in Anwesenheit eines die Termination unterdrükkenden Mittels bei Bedingungen, die Expression des Polypeptids und des Zellmembran-Ankerpeptids erlauben, wobei das die Termination unterdrückende Mittel ein Aminoglycosid-Antibiotikum ist,
c) Verwenden von Durchflußzytometrie, um mindestens eine Zelle auszuwählen, die ein Fusionsprotein exprimiert, umfassend das Polypeptid, das Zellmembran-Ankerpeptid und ein Reporterpeptid oder eine Epitop-Markierung, und
d) Kultivieren der ausgewählten Zelle in Abwesenheit eines die Termination unterdrückenden Mittels, um Expression eines Proteins, umfassend das Polypeptid in membrangebundener Form ohne das Reporterpeptid oder die Epitop-Markierung zu erhalten.

10. Verfahren nach Anspruch 9, wobei das Zellmembran-Ankerpeptid ein GPI-Anker ist.

11. Verfahren nach Anspruch 9 oder 10, wobei das zweite Polynukleotid ein Reporterpeptid kodiert, ausgewählt aus der Gruppe, bestehend aus grün fluoreszierendem Protein, Luciferase, β-Galactosidase, β-Glucuronidase und Chloramphenicol-Acetyltransferase (CAT).

12. Verfahren nach Anspruch 9 oder 10, wobei das zweite Polynukleotid eine Epitop-Markierung kodiert, ausgewählt aus der Gruppe, bestehend aus V5, His, FLAG™, HA, c-Myc, VSV-G und HSV.

13. Verfahren zum Herstellen eines Polypeptids, umfassend das Kultivieren einer Zelllinie, die durch das Verfahren nach einem der vorhergehenden Ansprüche erhalten wurde, wobei die Zelllinie in Abwesenheit eines Aminoglycosid-Antibiotikums kultiviert wird, um Expression des Polypeptids zu erlauben und Isolieren des Polypeptids.

14. Verfahren nach Anspruch 13, wobei das Polypeptid ein lösliches Polypeptid ist, das in ein Kulturmedium sekretiert wird, und das Polypeptid aus dem Medium isoliert wird.

15. Kit, das geeignet ist, das Verfahren nach einem der vorhergehenden Ansprüche durchzuführen, umfassend eines oder mehrere von: (1) mindestens ein Kit-Bestandteil, umfassend eine Expressionskassette wie in einem der Ansprüche 1 bis 12 definiert, eine Zelle oder einen Expressionsvektor, umfassend die Expressionskassette, ein Aminoglycosid-Antibiotikum oder eine Zusammensetzung, umfassend mindestens einen solchen Bestandteil, (2) Anweisungen zum Durchführen eines Verfahrens wie in einem der Ansprüche 1 bis 14 definiert, Anweisungen zum Verwenden eines in (1) bezeichneten Bestandteils oder einer Zusammensetzung jeglicher solcher Komponenten, (3) einen Behälter zur Aufbewahrung mindestens eines solchen Bestandteils oder einer solchen Zusammensetzung und (4) Verpackungsmaterial.

## Revendications

1. Procédé de criblage ou de sélection de cellules exprimant un niveau souhaité d'un polypeptide, comprenant les étapes consistant à :
a) fournir une pluralité de cellules eucaryotes comprenant chacune une cassette d'expression qui comprend un premier polynucléotide codant pour le polypeptide, au moins un codon d'arrêt en aval du premier polynucléotide, et un second polynucléotide codant pour un peptide d'ancrage à la membrane cellulaire en aval du codon d'arrêt ;
b) cultiver les cellules en présence d'un agent de suppression de la terminaison dans des conditions qui permettent l'expression du polypeptide, où l'agent de suppression de la terminaison est un antibiotique aminoglycoside ; et
c) utiliser la cytométrie de flux pour sélectionner au moins une cellule exprimant le polypeptide fusionné à un peptide d'ancrage à la membrane cellulaire.

2. Procédé d'évaluation de l'expression d'un polypeptide recombinant dans une population de cellules, comprenant les étapes consistant à :
a) fournir une pluralité de cellules eucaryotes comprenant chacune une cassette d'expression qui comprend un premier polynucléotide codant pour un polypeptide recombinant, au moins un codon d'arrêt en aval du premier polynucléotide, et un second polynucléotide codant pour un peptide d'ancrage à la membrane cellulaire en aval du codon d'arrêt ;
b) cultiver les cellules en présence d'un agent de suppression de la terminaison dans des conditions qui permettent l'expression d'une protéine de fusion comprenant le polypeptide recombinant et le peptide d'ancrage à la membrane cellulaire, où l'agent de suppression de la terminaison est un antibiotique aminoglycoside ; et
c) trier les cellules par cytométrie de flux pour sélectionner au moins une cellule exprimant la protéine de fusion à un niveau désiré et/ou selon une uniformité souhaitée.

3. Procédé de criblage ou de sélection d'au moins une cellule exprimant un polypeptide ayant une affinité de liaison souhaitée pour un ligand à partir de cellules exprimant une banque de variants polypeptidiques, comprenant les étapes consistant à :
a) fournir une pluralité de cellules eucaryotes comprenant chacune une cassette d'expression qui comprend un premier polynucléotide codant pour un variant polypeptidique, au moins un codon d'arrêt en aval du premier polynucléotide, et un second polynucléotide codant pour un peptide d'ancrage à la membrane cellulaire en aval du codon d'arrêt ;
b) cultiver les cellules en présence d'un agent de suppression de la terminaison dans des conditions qui permettent l'expression du variant polypeptidique, où l'agent de suppression de la terminaison est un antibiotique aminoglycoside ; et
c) utiliser une cytométrie de flux pour sélectionner au moins une cellule exprimant le variant polypeptidique fusionné à un peptide d'ancrage à la membrane cellulaire sur la base de l'affinité de liaison dudit variant polypeptidique audit ligand.

4. Procédé selon l'une quelconque des revendications précédentes, comprenant en outre l'étape consistant à :
d) cultiver au moins une cellule sélectionnée en l'absence d'un agent de suppression de la terminaison pour obtenir l'expression du polypeptide sous la forme d'un polypeptide soluble.

5. Procédé permettant d'exprimer alternativement i) un polypeptide soluble ou ii) un polypeptide lié à une membrane à partir d'une cellule unique ou d'une lignée cellulaire, comprenant les étapes consistant à :
a) fournir une pluralité de cellules eucaryotes comprenant chacune une cassette d'expression qui comprend un premier polynucléotide codant pour le polypeptide, au moins un codon d'arrêt en aval du premier polynucléotide, et un second polynucléotide codant pour un peptide d'ancrage à la membrane cellulaire en aval du codon d'arrêt ;
b) cultiver les cellules en présence d'un agent de suppression de la terminaison dans des conditions qui permettent l'expression du polypeptide, où l'agent de suppression de la terminaison est un antibiotique aminoglycoside ; et
c) utiliser la cytométrie de flux pour sélectionner au moins une cellule exprimant le polypeptide fusionné à un peptide d'ancrage à la membrane cellulaire ; et
d) cultiver ladite cellule sélectionnée en absence d'un agent de suppression de la terminaison pour obtenir l'expression du polypeptide sous la forme d'un polypeptide soluble.

6. Procédé selon l'une quelconque des revendications précédentes, dans lequel au moins une cellule sélectionnée exprime une protéine de fusion comprenant le polypeptide fusionné à un peptide d'ancrage à la membrane cellulaire, la protéine de fusion étant exposée à la surface de ladite cellule.

7. Procédé selon la revendication 6, dans lequel le peptide d'ancrage à la membrane cellulaire est une ancre GPI.

8. Procédé selon l'une quelconque des revendications précédentes, dans lequel l'antibiotique aminoglycoside est sélectionné dans le groupe constitué par le G-418, la gentamycine, la paromomycine, l'hygromycine, l'amikacine, la kanamycine, la néomycine, la nétilmicine, la paromycine, la streptomycine et la tobramycine.

9. Procédé d'expression en alternance de i) un polypeptide non marqué lié à une membrane ou ii) un polypeptide marqué lié à une membrane à partir d'une cellule unique ou d'une lignée cellulaire, comprenant les étapes consistant à :
a) fournir une pluralité de cellules eucaryotes comprenant chacune une cassette d'expression qui comprend un premier polynucléotide codant pour le polypeptide et un peptide d'ancrage à la membrane cellulaire, au moins un codon d'arrêt en aval du premier polynucléotide, et un second polynucléotide codant pour un peptide reporter ou un marqueur épitopique en aval du codon d'arrêt ;
b) cultiver les cellules en présence d'un agent de suppression de la terminaison dans des conditions qui permettent l'expression du polypeptide et du peptide d'ancrage à la membrane cellulaire, où l'agent de suppression de la terminaison est un antibiotique aminoglycoside ;
c) utiliser une cytométrie de flux pour sélectionner au moins une cellule exprimant une protéine de fusion qui comprend le polypeptide, le peptide d'ancrage à la membrane cellulaire et un peptide reporter ou un marqueur épitopique ; et
d) cultiver ladite cellule sélectionnée en absence d'un agent de suppression de la terminaison pour obtenir l'expression d'une protéine comprenant le polypeptide sous une forme liée à la membrane sans le peptide reporter ou le marqueur épitopique.

10. Procédé selon la revendication 9, dans lequel le peptide d'ancrage à la membrane cellulaire est une ancre GPI.

11. Procédé selon la revendication 9 ou 10, dans lequel le second polynucléotide code pour un peptide reporter sélectionné dans le groupe constitué par la protéine à fluorescence verte, de la luciférase, la β-galactosidase, la β-glucoronidase et la chloramphénicol acétyltransférase (CAT).

12. Procédé selon la revendication 9 ou 10, dans lequel le second polynucléotide code pour un marqueur épitopique sélectionné dans le groupe constitué par V5, His, FLAG™, HA, c-Myc, VSV-G et HSV.

13. Procédé de production d'un polypeptide, comprenant les étapes consistant à cultiver une lignée cellulaire obtenue par le procédé de l'une quelconque des revendications précédentes, dans lequel la lignée cellulaire est cultivée en l'absence d'un antibiotique aminoglycoside pour permettre l'expression du polypeptide, et à isoler ledit polypeptide.

14. Procédé selon la revendication 13, dans lequel le polypeptide est un polypeptide soluble qui est sécrété dans un milieu de culture, et dans lequel le polypeptide est isolé depuis ledit milieu.

15. Kit approprié pour exécuter le procédé selon l'une quelconque des revendications précédentes, comprenant un ou plusieurs des éléments suivants : (1) au moins un composant de kit comportant une cassette d'expression telle que définie dans l'une quelconque des revendications 1 à 12 ; une cellule ou un vecteur d'expression comprenant ladite cassette d'expression ; un antibiotique aminoglycoside ; ou une composition comprenant au moins un tel composant ; (2) des instructions permettant de réaliser un procédé tel que défini dans l'une quelconque des revendications 1 à 14, des instructions d'utilisation de l'un quelconque des composants identifiés en (1) ou de toute composition de n'importe lequel de ces composants ; (3) un conteneur pour loger ledit au moins un composant ou composition, et (4) des matériaux d'emballage.
